# EUROPEAN PATENT SPECIFICATION

(11) **EP 1 194 447 B1**
(45) Date of publication and mention of the grant of the patent: **26.09.2007**
(21) Application number: 00931037.6
(22) Date of filing: 29.05.2000
(51) Int. Cl.: C07K 14/705, C12N 15/12, C07K 16/28

(54) **NOVEL POTASSIUM CHANNELS AND GENES ENCODING THESE POTASSIUM CHANNELS**
NEUE KALIUM-KANÄLE UND DAFÜR KODIERENDE GENE
NOUVEAUX CANAUX POTASSIQUES ET GENES CODANT CES CANAUX POTASSIQUES

(30) Priority: 11.06.1999 DK 82899
(43) Date of publication of application: 10.04.2002
(73) Proprietor: NEUROSEARCH A/S, 2750 Ballerup (DK)
(72) Inventor: JENTSCH, Thomas, J., Universität Hamburg, D-20251 Hamburg (DE)
(86) International application number: PCT/DK2000/000289
(87) International publication number: WO 2000/077035

(56) References cited:
- WO-A-99/07832
- WO-A-99/21875
- DATABASE EMBL/GENBANK [Online] 14 May 2000 (2000-05-14) SCHROEDER B C ET AL: "KCNQ5, a novel potassium channel broadly expressed in brain, mediates M-type currents" Database accession no. AF202977 XP002901374
- KUBISCH CH ET AL: "KCNQ4, a novel potassium channel expressed in sensory outer hair cells, is mutated in dominant deafness" CELL, vol. 96, no. 5, 5 February 1999 (1999-02-05), pages 437-446, XP002901375
- DATABASE MEDLINE [Online] US NATIONAL LIBRARY OF MEDICINE (NLM), BETHESDA, MD, US; TAM S W ET AL: "Linopirdine. A depolarization-activated releaser of transmitters for treatment of dementia" Database accession no. 95343820 XP002900987 & ADV EXP MED BIOL, no. 363, 1995, pages 47-56, ISSN: 0065-2598

## Description

### TECHNICAL FIELD

This invention relates to novel potassium channels and genes encoding these channels. More specifically the invention provides isolated polynucleotides encoding the KCNQ5 potassium channel subunit, cells transformed with these polynucleotides, transgenic non-human animals comprising genetic mutations, and the use of the transformed cells for the *in vitro* screening of chemical compounds affecting KCNQ5 subunit containing potassium channels.

### BACKGROUND ART

Potassium channels participate in the regulation of electrical signalling in excitable cells, and regulate the ionic composition of biological fluids. Mutations in the four known genes of the *KCNQ* branch of the K⁺-channel gene family underlie inherited cardiac arrhythmia's, in some cases associated with deafness, neonatal epilepsy, and the progressive hearing loss of the elderly (presbyacusis).

Ion channels play important roles in signal transduction and in the regulation of the ionic composition of intra- and extracellular fluids. KCNQ1 is a typical member of the voltage-gated potassium channel superfamily with 6 transmembrane domains and a pore region situated between the fifth and the sixth transmembrane domain. The minK protein (also known as KCNE1 or IsK) has a single transmembrane span and cannot form potassium channels on its own. However, as a β-subunit it enhances and modifies currents mediated by KCNQ1. These heteromeric channels participate in the repolarization of the heart action potential. Certain mutations in either *KCNQ1* or *KCNE1* cause a form of the autosomal dominant long QT syndrome (LQTS), a disease characterised by repolarization anomalies of cardiac action potentials resulting in arrhythmias and sudden death. Interestingly, other mutations in either gene lead to the recessive Jervell and Lange-Nielsen (JLN) syndrome that combines LQTS with congenital deafness. In order to cause deafness, KCNQ1/minK currents must be reduced below levels that are already sufficiently low to cause cardiac arrhythmia.

Mutated and non-mutated KCNQ2 and KCNQ3 potassium channels have been disclosed in WO 99/07832, WO 99/21875 and WO 99/31232.

### SUMMARY OF THE INVENTION

We have now cloned and characterised KCNQ5, a novel member of the KCNQ family of potassium channel proteins. KCNQ5 forms heteromeric channels with other KCNQ channel subunits, in particular KCNQ3 and KCNQ4.

The present invention has important implications for the characterisation and exploitation of this interesting branch of the potassium channel super family.

Accordingly, in its first aspect, the invention provides an isolated polynucleotide having a nucleic acid sequence which is capable of hybridising under at least medium/high stringency conditions with the polynucleotide sequence presented as SEQ ID NO: 1, or its complementary strand, wherein said hybridization occurs in a solution of 5 x SSC, 5 x Denhardt's solution, 0.5 % SDS and 100 µg/ml of denatured sonicated salmon sperm DNA for 12 hours at approximately 45°C, followed by washing twice for 30 minutes in 2 x SSC, 0.5 % SDS at a temperature of at least 65°C, and encoding a potassium channel subunit.

In another aspect the invention provides a recombinantly produced polypeptide encoded by the polynucleotide of the invention.

In a third aspect the invention provides a cell genetically manipulated by the incorporation of a heterologous polynucleotide of the invention.

In a fourth aspect the invention provides a method of screening a chemical compound for inhibiting or activating or otherwise modulating the activity on a potassium channel comprising at least one KCNQ5 channel subunit of the invention, which method comprises the steps of subjecting a KCNQ5 channel subunit containing cell of the invention to the action of the chemical compound; and monitoring the membrane potential, the current, the potassium flux, or the secondary calcium influx of the KCNQ5 channel subunit containing cell.

In a fifth aspect the invention relates to the use of a polynucleotide sequence of the invention for the screening of genetic materials from humans suffering from neurological diseases for mutations in the *KCNQ5* gene.

In a sixth aspect the invention provides a transgenic animal other than a human, comprising a knock-out mutation of the endogenous *KCNQ5* gene, a replacement by or an additional expression of a mutated *KCNQ5* gene, or genetically manipulated in order to over-express the *KCNQ5* gene or to over-express mutated *KCNQ5* gene.

Other objects of the invention will be apparent to the person skilled in the art from the following detailed description and examples.

### DETAILED DISCLOSURE OF THE INVENTION

The present invention provides novel potassium channels and genes encoding these channels. The invention also provides cells transformed with these genes, transgenic animals other than a human comprising genetic mutations, and the use of the transformed cells for the *in vitro* and *in vivo* screening of drugs affecting KCNQ5 containing potassium channels.

### Polynucleotides

In its first aspect, the invention provides polynucleotides capable of hybridising under at least medium/high stringency conditions with the polynucleotide sequence presented as SEQ ID NO: 1, its complementary strand,wherein said hybridization occurs in a solution of 5 x SSC, 5 x Denhardt's solution, 0.5 % SDS and 100 µg/ml of denatured sonicated salmon sperm DNA for 12 hours at approximately 45°C, followed by washing twice for 30 minutes in 2 x SSC, 0.5 % SDS at a temperature of at least 65°C, and encoding a potassium channel subunit.

The polynucleotides of the invention include DNA, cDNA and RNA sequences, as well as anti-sense sequences, and include naturally occurring, synthetic, and intentionally manipulated polynucleotides. The polynucleotides of the invention also include sequences that are degenerate as a result of the genetic code.

As defined herein, the term "polynucleotide" refers to a polymeric form of nucleotides of at least 10 bases in length, preferably at least 15 bases in length. By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. The term therefore includes recombinant DNA which is incorporated into an expression vector, into an autonomously replicating plasmid or virus, or into the genomic DNA of a prokaryote or eukaryote, or which exists as a separate molecule, e.g. a cDNA, independent from other sequences.

The polynucleotides of the invention also include allelic variants and "mutated polynucleotides" having a nucleotide sequence that differs from the sequence presented as SEQ ID NO: 1 at one or more nucleotide positions. The mutated polynucleotide may in particular be a polynucleotide of the invention having a nucleotide sequence as in SEQ ID NO: 1, which sequence, however, differs from SEQ ID NO: 1 so as to effect the expression of a variant polypeptide. The mutated polynucleotide may be a polynucleotide of the invention having a nucleotide sequence encoding a potassium channel having an amino acid sequence that has been changed at one or more positions. The mutated polynucleotide may in particular be a polynucleotide of the invention having a nucleotide sequence encoding a potassium channel having an amino acid sequence that has been changed at one or more positions located in the conserved regions, as defined by Table 1, below.

### Hybridisation Protocol

The polynucleotides of the invention are such which have a nucleic acid sequence capable of hybridising with the polynucleotide sequence presented as SEQ ID NO: 1, its complementary strand, under at least medium/high, or high stringency conditions, as described in more detail below.

In a preferred embodiment the polynucleotide hybridise to DNA that encodes a polypeptide of the invention, preferably the polypeptide having the amino acid sequence presented as SEQ ID NO: 2 under at least medium/high, or high stringency conditions, as described in more detail below.

Suitable experimental conditions for determining hybridisation between a nucleotide probe and a homologous DNA or RNA sequence, involves pre-soaking of the filter containing the DNA fragments or RNA to hybridise in 5 x SSC [Sodium chloride/Sodium citrate; cf. Sambrook et al.; Molecular Cloning: A Laboratory Manual, Cold Spring Harbor Lab., Cold Spring Harbor, NY 1989] for 10 minutes, and pre-hybridisation of the filter in a solution of 5 x SSC, 5 x Denhardt's solution [cf. *Sambrook et al.; Op cit.*], 0.5 % SDS and 100 µg/ml of denatured sonicated salmon sperm DNA [cf. *Sambrook et al.; Op cit.*], followed by hybridisation in the same solution containing a concentration of 10 ng/ml of a random-primed [Feinberg A P & Vogelstein B; Anal. Biochem. 1983 132 6-13], ³²p-dCTP-labeled (specific activity > 1 x 10⁹ cpm/µg) probe for 12 hours at approximately 45°C.

The filter is then washed twice for 30 minutes in 2 x SSC, 0.5 % SDS at a temperature of at least at least 65°C (medium/high stringency conditions), more preferred of at least 70°C (high stringency conditions), and even more preferred of at least 75°C (very high stringency conditions).

Molecules to which the oligonucleotide probe hybridises under these conditions may be detected using a x-ray film.

### DNA Sequence Homology

In a preferred embodiment, the polynucleotides of the invention show a degree of identity of at least 90%, most preferred at least 95%, with the polynucleotide sequence presented as SEQ ID NO: 1.

As defined herein, the DNA sequence homology may be determined as the degree of identity between two DNA sequences indicating a derivation of the first sequence from the second. The homology may suitably be determined by means of computer programs known in the art such as GAP provided in the GCG program package [Needleman S B and Wunsch C D, Journal of Molecular Biology 1970 48 443-453] using default parameters suggested herein.

### Cloned Polynucleotides

The isolated polynucleotide of the invention may in particular be a cloned polynucleotide.

As defined herein, the term "cloned polynucleotide", refers to a polynucleotide or DNA sequence cloned in accordance with standard cloning procedures currently used in genetic engineering to relocate a segment of DNA, which may in particular be cDNA, i.e. enzymatically derived from RNA, from its natural location to a different site where it will be reproduced.

Cloning may be accomplished by excision and isolation of the desired DNA segment, insertion of the piece of DNA into the vector molecule and incorporation of the recombinant vector into a cell where multiple copies or clones of the DNA segment will be replicated, by reverse transcription of mRNA (reverse transcriptase technology), and by use of sequence-specific oligonucleotides and DNA polymerase in a polymerase chain reaction (PCR technology).

The cloned polynucleotide of the invention may alternatively be termed "DNA construct" or "isolated DNA sequence", and may in particular be a complementary DNA (cDNA).

It is well established that potassium channels may be formed as heteromeric channels, composed of different subunits. Also it has been established that the potassium channel of the invention may form heteromers with other KCNQ's, in particular KCNQ3 and KCNQ4, when co-expressed with these subunits. In addition, potassium channels can associate with non-homologous subunits (β-subunits), e.g. the KCNE1 (also known as minK or IsK), the KCNE2 (also known as the minK-related peptide (MiRP1), the KCNE3 (also known as MiRP2), the KCNE4 (also known as MiRP3), and/or the KCNE5 (also known as KCNE1L) subunit, that co-assemble and functionally modulate these channels or lead to a specific localisation within the cell.

Therefore, in a preferred embodiment, the polynucleotide of the invention is cloned and either expressed by itself or co-expressed with polynucleotides encoding other subunits, in particular a polynucleotide encoding a KCNQ3 channel subunit or a polynucleotide encoding a KCNQ4 channel subunit.

In another aspect of the invention, isolated and purified KCNQ5 antisence oligonucleotides can be made and a method utilised for diminishing the level of expression of KCNQ5 by a cell comprising administering one or more KCNQ5 antisense oligonucleotides. By KCNQ5 antisense oligonucleotides reference is made to oligonucleotides that have a nucleotide sequence that interacts through base pairing with a specific complementary nucleic acid sequence involved in the expression of KCNQ5 such that the expression of KCNQ5 is reduced. Preferably, the nucleic acid sequence involved in the expression of KCNQ5 is a genomic DNA molecule or mRNA molecule that encodes KCNQ5. This genomic DNA molecule can comprise regulatory regions of the KCNQ5 gene, the pre- or pro- portions of the *KCNQ5* gene, or the coding sequence for mature KCNQ5 protein.

The term "complementary to a nucleotide sequence" in the context of KCNQ5 antisense oligonucleotides and methods therefor means sufficiently complementary to such a sequence as to allow hybridization to that sequence in a cell, i.e. under physiological conditions. The KCNQ5 antisense oligonucleotides preferably comprise a sequence comprising of from about 8 to about 100 nucleotides, more preferably of from about 15 to about 30 nucleotides.

The KCNQ5 antisense oligonucleotides can also include derivatives which comprise a variety of modifications that confer resistance to nucleolytic degradation such as e.g. modified internucleoside linkages modified nucleic acid bases and/or sugars and the like. Examples of such derivatives include backbone modifications such as phosphotriester, phosphorothioate, methylphosphate, phosphoramidate, phosphorodithioate and formacetal as well as morpholino, peptide nucleic acid analogues and dithioate repeating units.

The usefulness of antisense molecules have been described by e.g. Toulme & Helene, Gene 1988 72 51-58; Inouye, Gene 1988 72 25-34; *Uhlmann &* Peyman, Chemical Reviews 1990 90 543-584; Robertson, Nature Biotechnology 1997 15 209; and Gibbons & Dzau, Science 1996 272 689-693.

### Biological Sources

The isolated polynucleotide of the invention may be obtained from any suitable human or animal source. In a preferred embodiment, which the polynucleotide of the invention is cloned from, or produced on the basis of a cDNA library, e.g. of the retina, skeletal muscle, or brain, in particular the cerebral cortex, occipital pole, frontal and temporal lobes, putamen and the hippocampus, and in the piriform cortex, the entorhinal cortex, the pontine medulla and the facial nucleus, and in the cerebellum. In a more preferred embodiment, which the polynucleotide of the invention is cloned from, or produced on the basis of a cDNA library of the thalamus. Commercial cDNA libraries are available from e.g. Stratagene and Clontech.

The KCNQ5 gene of the invention has been localised to the long arm of chromosome 6 (6q14).

The isolated polynucleotide of the invention may be obtained methods known in the art, e.g. those described in the working examples below.

### Preferred Polynucleotides

In a preferred embodiment, polynucleotide of the invention has the polynucleotide sequence presented as SEQ ID NO: 1.

In another preferred embodiment the polynucleotide of the invention is a sequence giving rise to KCNQ5 channels subunits comprising one or more substitutions.

In another preferred embodiment the polynucleotide of the invention is a sequence giving rise to KCNQ5 channels subunits comprising one or more substitutions in the conserved regions, as defined in more details below.

It has been demonstrated that KCNQ channels often show alternative splicing and therefore may occur as isoforms originating from the same gene. Such isoforms as well as the different cDNA sequences from which they occurred are also contemplated within the scope of the present invention.

Finally the genes encoding KCNQ channel subunits in other species have been found to differ slightly from the human genes. However, genes of other species, e.g. mouse, rat, monkey, rabbit, etc., are also contemplated within the scope of the present invention.

### Recombinantly Produced Polypeptides

In another aspect the invention relates to novel KCNQ5 proteins. More specifically, the invention relates to substantially pure functional polypeptides that have the electrophysiological and pharmacological properties of a KCNQ5 channel, or KCNQ5 channel subunits. The novel polypeptides of the invention may be obtained by the polynucleotides of the invention using standard recombinant DNA technology.

In a preferred embodiment, a polypeptide of the invention is the KCNQ5 potassium channel having the amino acid sequence presented as SEQ ID NO: 2.

Modifications of this primary amino acid sequence may result in proteins which have substantially equivalent activity as compared to the unmodified counterpart polypeptide, and thus may be considered functional analogous of the parent proteins. Such modifications may be deliberate, e.g. as by site-directed mutagenesis, or they may occur spontaneous, and include splice variants, isoforms, homologues from other species, and polymorphisms. Such functional analogous are also contemplated according to the invention.

Moreover, modifications of this primary amino acid sequence may result in proteins which do not retain the biological activity of the parent protein, including dominant negative forms, etc. A dominant negative protein may interfere with the wild-type protein by binding to, or otherwise sequestering regulating agents, such as upstream or downstream components, that normally interact functionally with the polypeptide.

In the context of this invention, the term "variant polypeptide" means a polypeptide (or protein) having an amino acid sequence that differs from the sequence presented as SEQ ID NO: 2 at one or more amino acid positions. Such variant polypeptides include the modified polypeptides described above, as well as conservative substitutions, splice variants, isoforms, homologues from other species, and polymorphisms.

As defined herein, the term "conservative substitutions" denotes the replacement of an amino acid residue by another, biologically similar residue. Examples of conservative substitutions include the substitution of one hydrophobic residue such as isoleucine, valine, leucine or methionine for another, or the substitution of one polar residue for another, such as the substitution of arginine for lysine, glutamic for aspartic acid, or glutamine for asparagine, and the like. The term conservative substitution also include the use of a substituted amino acid residue in place of an un-substituted parent amino acid residue provided that antibodies raised to the substituted polypeptide also immune-react with the un-substituted polypeptide.

### KCNQ1 Numbering System

In the context of this invention, amino acid residues (as well as nucleic acid bases) are specified using the established one-letter symbol.

By aligning the amino acid sequences of a polypeptide of the present invention to those of the known polypeptides, a specific amino acid numbering system may be employed, by which system it is possible to unambiguously allot an amino acid position number to any amino acid residue in any KNCQ channel protein, which amino acid sequence is known.

Such an alignment is presented in Table 1, below. Using the ClustalX computer alignment program [Thompson JD, Gibson TJ, Plewniak F, Jeanmougin F, & Higgins DG: The ClustalX windows interface: flexible strategies for multiple sequence alignment aided by quality analysis tools; Nucleic Acids Res. 1997 25 (24) 4876-82], and the default parameters suggested herein, the amino acid sequence of a polypeptide of the present invention (hKCNQ5) and the amino acid sequences of the known polypeptides hKCNQ2-4 are aligned with, and relative to, the amino acid sequences of the known polypeptide hKCNQ1 (also known as KvLQT1). In the context of this invention this numbering system is designated the KCNQ1 Numbering System.

In describing the various enzyme variants produced or contemplated according to the invention, the following nomenclatures have been adapted for ease of reference:

### Original amino acid / Position / Substituted amino acid

According to this nomenclature the substitution of serine for glycine at position 329 of Table 1 is designated as "G329S".

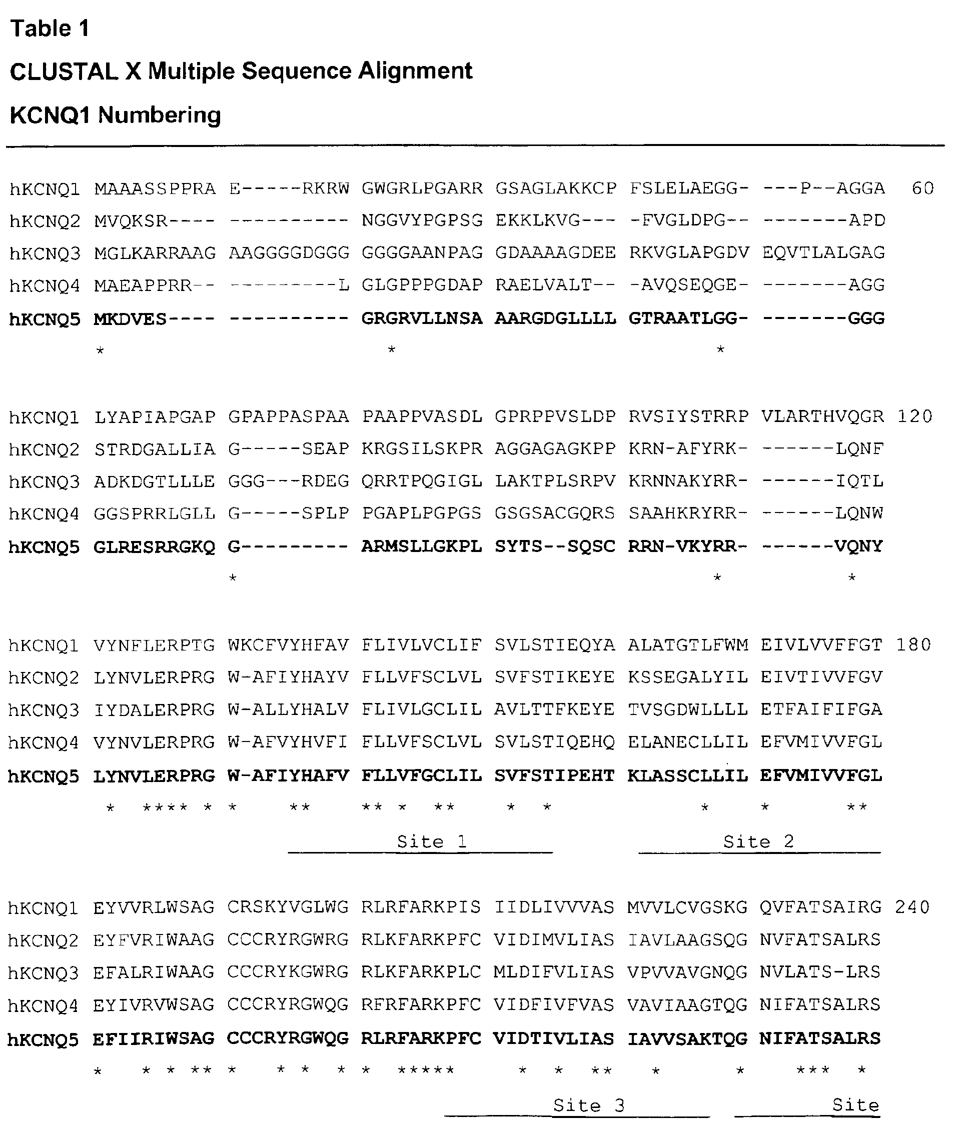

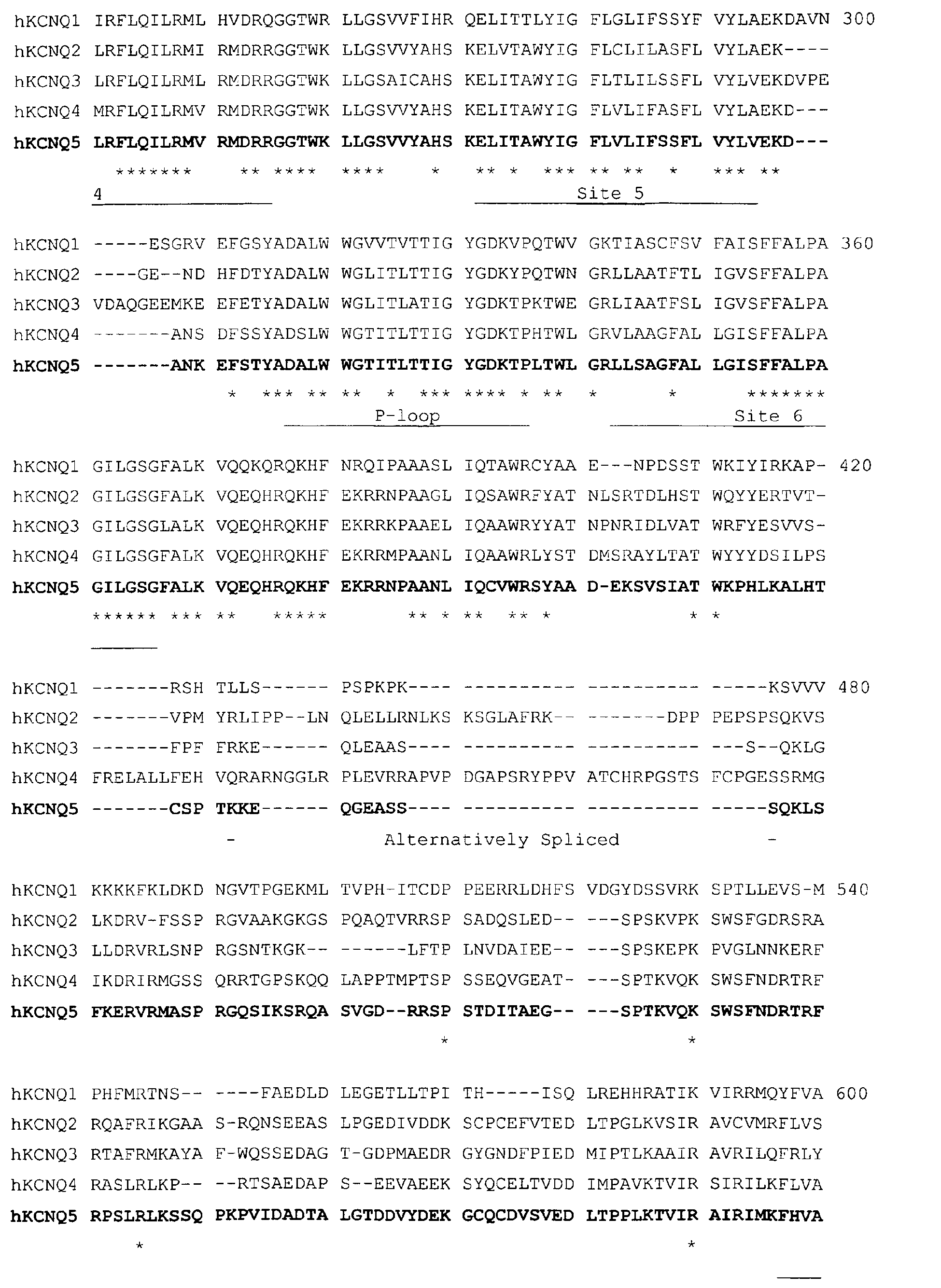

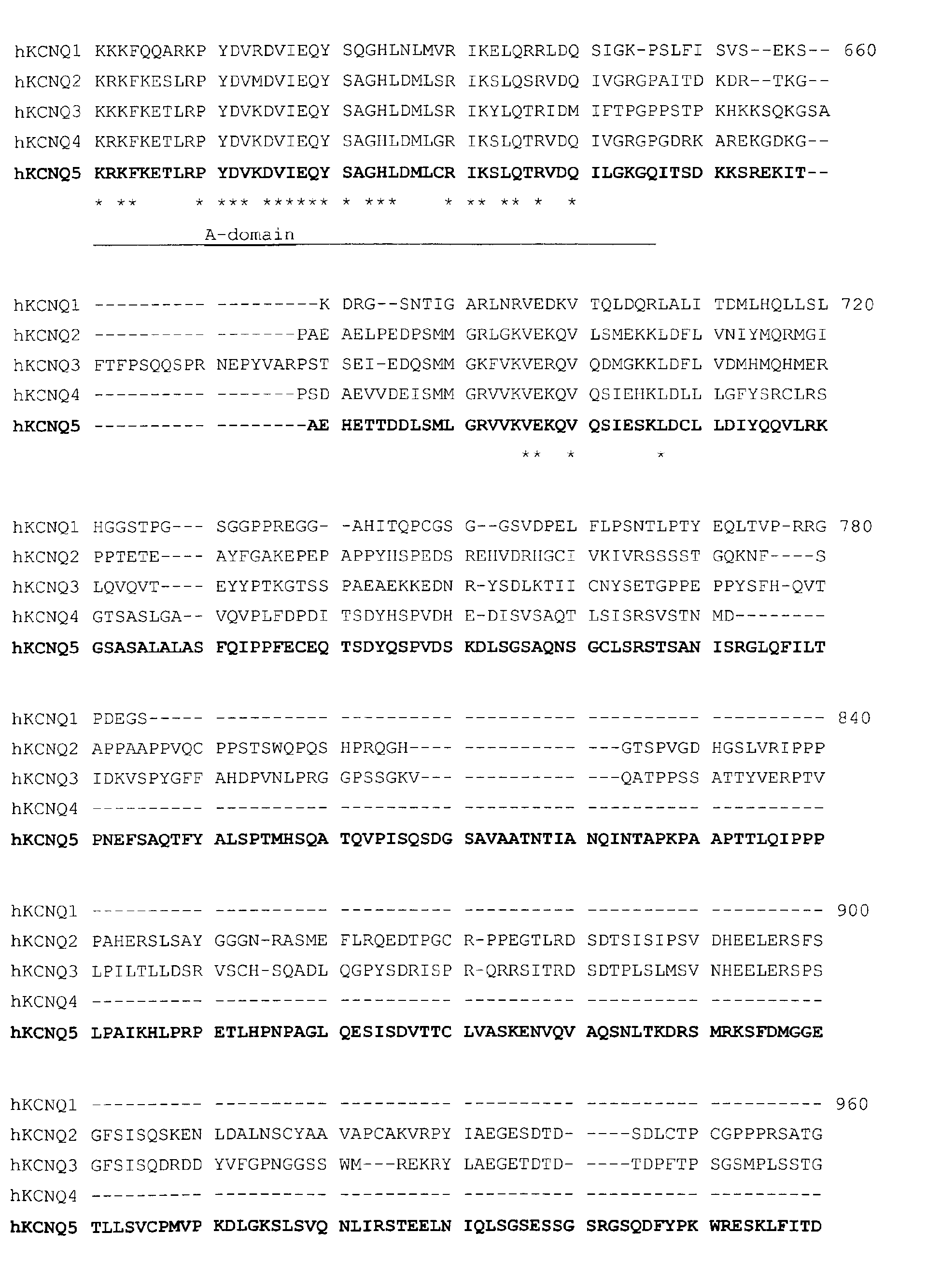

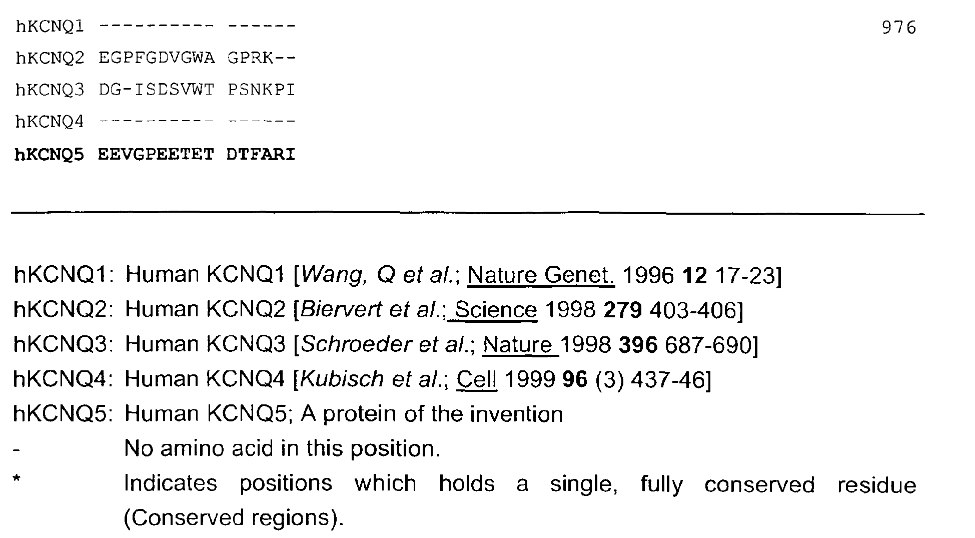

Preferred variants are the splice variants at positions 432-476 (KCNQ1 Numbering) holding the following amino acid residues:
1) KKE----- QGEASS---- ------NKFC SNKQKLFRMY TSRKQS;
2)KKE----- QGEASS---- ----- ----- ----- ;
3) ----- ----- ----- ----- ----- ; or
4)--------- ---------- ------NKFC SNKQKLFRMY TSRKQS.

Another preferred variants is G329S (KCNQ1 numbering), or KCNQ5/G278S ("KCNQ5 numbering").

### Biological Activity

Ion channels are excellent targets for drugs. The polynucleotide of the invention encodes a potassium channel, which has been termed KNCQ5.

KCNQ5, or heteromeric channels containing the KCNQ5 subunit, may be a particularly interesting target for the treatment of diseases or adverse conditions of the CNS, including affective disorders, Alzheimer's disease, anxiety, ataxia, CNS damage caused by trauma, stroke or neurodegenerative illness, cognitive deficits, compulsive behaviour, dementia, depression, Huntington's disease, learning deficiencies, mania, memory impairment, memory disorders, memory dysfunction, motion disorders, motor disorders, motor neuron doseases, myokymia, neurodegenerative diseases, Parkinson's disease and Parkinson-like motor disorders, phobias, Pick's disease, psychosis, schizophrenia, seizures, incl. epileptic seizures, spinal cord damage, stroke, tremor, seizures, convulsions and epilepsy.

The novel polynucleotides of the invention may in itself be used as a therapeutic or diagnostic agent. For gene therapy, the person skilled in the art may use sence or antisense nucleic acid molecules as therapeutic agents for KCNQ-related indications.

### Heteromers Formed by KCNQ Subunits

The KCNQ channels described so far function physiologically as heteromers. KCNQ1 associates with KCNE1 (also known as minK or IsK); KCNQ2 and KCNQ3 form heteromeric channels that underlie the M-current, an important determinant of neuronal excitability that is regulated by several neurotransmitters, and KCNQ4 is supposed to combine with KCNQ3 to mediate the I_{M}-like current in the outer hair cells.

Like other KCNQ channel subunits, KCNQ5 may interact with other subunits, e.g. KCNE1 or other KCNQ channel subunits, and in particular with KCNQ3, and with KCNQ4. Currents from homomeric KCNQ3 are very small and often cannot be distinguished from *Xenopus* oocyte background currents. Co-expression of KCNQ3 with KCNQ5 markedly increased current amplitudes. Co-expression of KCNQ4 with KCNQ5 markedly decreased current amplitudes.

### Antibodies

The polypeptides of the invention can be used to produce antibodies which are immunoreactive or bind to epitopes of these polypeptides. Polyclonal antibodies which consist essentially of pooled monoclonal antibodies with different specificities, as well as distinct monoclonal antibody preparations may be provided. Polyclonal antibodies which are made up of pooled monoclonal antibodies with different specificities, as well as distinct monoclonal antibody preparations may be provided.

The preparation of polyclonal and monoclonal antibodies is well known in the art. Polyclonal antibodies may in particular be obtained as described by e.g. Green et al.: "Production of Polyclonal Antisera" in Immunochemical Protocols (Manson, Ed.); Humana Press, 1992, Pages 1-5; Coligan et al.: "Production of Polyclonal Antisera in rabbits, rats, Mice and Hamsters" in Current Protocols in Immunology, 1992, Section 2.4.1; and Ed Harlow and David Lane (Eds.) in "Antibodies; A laboratory manual", Cold Spring Harbor Lab. Press 1988; which protocols are hereby incorporated by reference.

Monoclonal antibodies may in particular be obtained as described by e.g. Kohler & Milstein, Nature 1975 256 495; Coligan et al. in Current Protocols in Immunology, 1992, Sections 2.5.1 - 2.6.7; Harlow et al. in Antibodies: A Laboratory Manual; Cold Spring Harbor Pub., 1988, Page 726; which protocols are hereby incorporated by reference.

Briefly, monoclonal antibodies may be obtained by injecting e.g. mice with a composition comprising an antigen, verifying the presence of antibody production by removing a serum sample, removing the spleen to obtain B lymphocytes, fusing the B lymphocytes with myeloma cells to produce hybridomas, cloning the hybridomas, selecting positive clones that produce the antibodies to the antigen, and isolating the antibodies from the hybridoma cultures.

Monoclonal antibodies can be isolated and purified from hybridoma cultures by a variety of well-established techniques, including affinity chromatography with protein A Sepharose, size-exclusion chromatography, and ion-exchange chromatography, see. e.g. Coligan et al. in Current Protocols in Immunology, 1992, Sections 2.7.1 - 2.7.12, and Sections 2.9.1 - 2.9.3; and Barnes et al.: "Purification of Immunoglobulin G (IgG)" in Methods in Molecular Biology; Humana Press, 1992, Vol. 10, Pages 79-104.

The polyclonal or monoclonal antibodies may optionally be further purified, e.g. by binding to and elution from a matrix to which the polypeptide, to which the antibodies were raised, is bound.

Antibodies which bind to the polypeptide of the invention can be prepared using an intact polypeptide or fragments containing small peptides of interest as the immunising antigen. The polypeptide used to immunise an animal may be obtained by recombinant DNA techniques or by chemical synthesis, and may optionally be conjugated to a carrier protein. Commonly used carrier proteins which are chemically coupled to the peptide include keyhole limpet hemocyanin (KLH), thyroglobulin, bovine serum albumin (BSA), and tetanus toxoid. The coupled peptide may then be used to immunise the animal, which may in particular be a mouse, a rat, a hamster or a rabbit.

### Genetically Manipulated Cells

In a third aspect the invention provides a cell genetically manipulated by the incorporation of the heterologous polynucleotide of the invention. The cell of the invention may in particular be genetically manipulated to transiently or stably express, over-express or co-express a KCNQ5 channel subunit as defined above. Methods of transient and stable transfer are known in the art.

The polynucleotide of the invention may be inserted into an expression vector, e.g. a plasmid, virus or other expression vehicle, and operatively linked to expression control sequences by ligation in a way that expression of the coding sequence is achieved under conditions compatible with the expression control sequences. Suitable expression control sequences include promoters, enhancers, transcription terminators, start codons, splicing signals for introns, and stop codons, all maintained in the correct reading frame of the polynucleotide of the invention so as to permit proper translation of mRNA. Expression control sequences may also include additional components such as leader sequences and fusion partner sequences.

The promoter may in particular be a constitutive or an inducible promoter. When cloning in bacterial systems, inducible promoters such as pL of bacteriophage γ, plac, ptrp, ptac (ptrp-lac hybrid promoter), may be used. When cloning in mammalian systems, promoters derived from the genome of mammalian cells, e.g. the TK promoter or the metallothionein promoter, or from mammalian viruses, e.g. the retrovirus long terminal repeat, the adenovirus late promoter or the vaccinia virus 7.5K promoter, may be used. Promoters obtained by recombinant DNA or synthetic techniques may also be used to provide for transcription of the polynucleotide of the invention.

Suitable expression vectors typically comprise an origin of expression, a promoter as well as specific genes which allow for phenotypic selection of the transformed cells, and include vectors like the T7-based expression vector for expression in bacteria [Rosenberg et al; Gene 1987 56 125], the pMSXND expression vector for expression in mammalian cells [Lee and Nathans, J. Biol. Chem. 1988 263 3521], baculovirus derived vectors for expression in insect cells, and the oocyte expression vector PTLN [Lorenz C, Pusch M & Jentsch T J: Heteromultimeric CLC chloride channels with novel properties; Proc. Natl. Acad. Sci. USA 1996 93 13362-13366].

In a preferred embodiment, the cell of the invention is an eukaryotic cell, in particular a mammalian cell, an oocyte, or a yeast cell. In a more preferred embodiment, the cell of the invention is a human embryonic kidney (HEK) cell, a HEK 293 cell, a BHK21 cell, a Chinese hamster ovary (CHO) cell, a *Xenopus laevis* oocyte (XLO) cell, a COS cell, or any other cell line able to express KCNQ potassium channels.

When the cell of the invention is an eukaryotic cell, incorporation of the heterologous polynucleotide of the invention may be in particular be carried out by infection (employing a virus vector), by transfection (employing a plasmid vector), or by calcium phosphate precipitation, microinjection, electroporation, lipofection, or other physical-chemical methods known in the art.

In a further preferred embodiment, the cell of the invention is genetically manipulated to co-express KCNQ5 and KCNQ1 channel subunits; KCNQ5 and KCNQ2 channel subunits; KCNQ5 and KCNQ3 channel subunits; KCNQ5 and KCNQ4 channel subunits; KCNQ5 and KCNQ1 and KCNQ2 channel subunits; KCNQ5 and KCNQ1 and KCNQ3 channel subunits; KCNQ5 and KCNQ2 and KCNQ3 channel subunits; KCNQ5 and KCNQ1 and KCNQ4 channel subunits; KCNQ5 and KCNQ2 and KCNQ4 channel subunits; KCNQ5 and KCNQ3 and KCNQ4 channel subunits; KCNQ5 and KCNQ1 and KCNQ2 and KCNQ3 channel subunits; KCNQ5 and KCNQ1 and KCNQ2 and KCNQ4 channel subunits; KCNQ5 and KCNQ1 and KCNQ3 and KCNQ4 channel subunits, or KCNQ5 and KCNQ2 and KCNQ3 and KCNQ4 channel subunits.

In another preferred embodiment, membrane preparations are provided. The membrane preparations of the invention may typically be used for screening purposes, and may be obtained by standard techniques.

In a preferred embodiment, frozen intact cells of the invention are homogenised while in cold water suspension and a membrane pellet is collected after centrifugation. The pellet may then be washed in cold water and dialysed to remove endogenous ligands that could compete for binding in the assays. The dialysed membranes may be used as such, or after storage in lyophilised form.

### Screening of Drugs

In a further aspect the invention provides methods for screening for KCNQ5 active compounds, i.e. chemical compounds capable of binding to, and showing activity at potassium channels containing one or more KCNQ5 subunits. The activity determined may be inhibitory activity, stimulating activity, or other modulatory activity. In particular the KCNQ5 active compound may induce a second messenger response, which cause a change of the molecular characteristics of the cell, e.g. the ion flux, enzyme activation, changes in the level of intracellular Ca²⁺ or H⁺, changes cyclic nucleotides such as cAMP, cADP, cGMP, and cGDP, etc.

Therefore, in another aspect, the invention provides a method for identifying functional ligands for a human potassium channel, comprising a KCNQ5 subunit, which method comprises transfecting cells with one or more polypeptides of the invention, encoding a KCNQ5 channel subunit of the invention, and detecting the effect on the signal transduction pathway caused in these cells by binding of the ligands to the receptor by a reporter system.

Such chemical compounds can be identified by one of, or both methods described below.

### Binding Studies

Binding studies are usually carried out by subjecting the target to binding with a labelled, selective agonist (binding agent), to form a labelled complex, followed by determination of the degree of displacement caused by the test compound upon addition to the complex.

In a specific aspect the invention provides a method of screening a chemical compound for capability of binding to a potassium channel comprising at least one KCNQ5 channel subunit of the invention, which method comprises the steps of (i) subjecting a KCNQ5 channel subunit containing cell of the invention to the action of a KCNQ5 binding agent to form a complex with the KCNQ5 channel subunit containing cell; (ii) subjecting the complex of step (i) to the action of the chemical compound to be tested; and (iii) detecting the displacement of the KCNQ5 binding agent from the complex with the KCNQ5 channel subunit containing cell.

The KCNQ5 channel subunit containing cell preferably is a cell of the invention as described above.

The KCNQ5 binding agent preferably is a radioactively labelled 1,3-dihydro-1-phenyl-3,3-bis-(4-pyridylmethyl)-2H-indol-2-one (Linopirdine); or 10,10-bis-(4-pyridinyl-methyl)-9-(10H)-antracenone (XE991).

In a even more preferred embodiment, the binding agent is labelled with ³H, and the displacement of the KCNQ5 binding agent from the complex with the KCNQ5 channel subunit containing cell is detected by measuring the amount of radioactivity by conventional liquid scintillation counting.

### Activity Studies

The KCNQ5 channel agonists may affect the potassium channel in various ways. The agonist may in particular show inhibitory activity, stimulating activity, or other modulatory activity.

In a specific aspect the invention provides a method for determining the activity at potassium channels containing one or more KCNQ5 subunits. According to this method a KCNQ5 channel subunit containing cell of the invention is subjecting to the action of the chemical compound to be tested, and the activity is detected by way of monitoring the membrane potential, the current, the potassium flux, or the secondary calcium influx of the KCNQ5 channel subunit containing cell, preferably a genetically manipulated as described above.

The membrane potential and the current may be monitored by electrophysiologic methods, including patch clamp techniques, such as current clamp technology and two-electrode voltage clamp technology, or by spectroscopic methods, such as fluorescence methods.

In a preferred embodiment, monitoring of the membrane potential of the KCNQ5 channel subunit containing cell is performed by patch clamp techniques.

In another preferred embodiment, monitoring of the membrane potential of the KCNQ5 channel subunit containing cell is performed by spectroscopic methods, e.g. using fluorescence methods. In a more specific embodiment, the KCNQ5 channel subunit containing cell is mixed with a membrane potential indicating agent, that allow for a determination of changes in the membrane potential of the cell, caused by the addition of the test compound. The membrane potential indicating agent may in particular be a fluorescent indicator, preferably DIBAC₄(3), DiOC5(3), and DiOC2(3).

In yet a preferred embodiment, monitoring of the membrane potential of the KCNQ5 channel subunit containing cell is performed by spectroscopic methods, e.g. using a FLIPR assay (Fluorescence Image Plate Reader; available from Molecular Devices).

### Screening of Genetic Material

In a further aspect the invention relates to the use of a polynucleotide sequence of the invention for the screening of genetic materials. By this method, individuals bearing a gene identical or homologous to a polynucleotide of the invention may be identified.

In the screening method of the invention, a polynucleotide of the invention, or any fragment or sub-sequence hereof, is employed. For the identification of individuals bearing mutated genes preferably the mutated forms of the polynucleotide represented by SEQ ID NO: 1 are employed.

In the screening method of the invention only short sequences needs to be employed depending on the actual method used. For SSCA, several hundreds of base pairs may be needed, for oligonucleotide or PCR hybridisation only of from about 10 to about 50 basepairs may be needed.

The screening may be accomplished by conventional methods, including hybridisation, SSCA analysis, and microarray technology (DNA chip technology). The hybridisation protocol described above represents a suitable protocol for use in a screening method of the invention.

### Transgenic Animals

Transgenic non-human animal models provide the means, *in vivo,* to screen for therapeutic compounds. Since KCNQ5 is expressed also in brain, they may be helpful in screening for drugs effective in CNS disorders, e.g. epilepsy.

By transgene is meant any piece of polynucleotide which is inserted by artifice into a cell, and thus becomes part of the genome of the organism that develops from that cell. Such a transgene may include a gene which is partly or entirely heterologous (i.e. foreign) to the transgenic organism, or it may represent a gene homologous to an endogenous gene of the organism.

By a transgenic animal is meant any non-human organism holding a cell which includes a polynucleotide sequence which is inserted into that cell by artifice, and which cell becomes part of the transgenic organism which develops from that cell. Such a transgene may be partly or entirely heterologous to the transgenic animal. Although transgenic mice represent a preferred embodiment of the invention, other transgenic mammals including transgenic rodents (e.g. hamsters, guinea pigs, rabbits and rats), and transgenic pigs, cattle, sheep and goats may be created by standard techniques and are included in the invention.

Preferably, the transgene is inserted by artifice into the nuclear genome.

### Knock-out and Knock-in Animals

The transgenic knock-out non-human animal models may be developed by homologous recombination of embryonic stem cells with constructs containing genomic sequence from the *KCNQ5* gene, that lead to a loss of function of the gene after insertion into the endogenous gene.

By knock-out mutation is meant an alteration in the polynucleotide sequence that reduces the biological activity of the polypeptide normally encoded therefrom. In order to create a true knock-out model, the biological activity of the expressed polypeptide should be reduced by at least 80% relative to the un-mutated gene. The mutation may in particular be a substitution, an insertion, a deletion, a frameshift mutation, or a mis-sense mutation. Preferably the mutation is a substitution, an insertion or a deletion.

To further assess the role of KCNQ5 at an organism level, the generation of an animal, preferably a mouse, lacking the intact *KCNQ5* gene, or bearing a mutated *KCNQ5* gene, is desired.

A replacement-type targeting vector, which may be used to create a knock-out model, may be constructed using an isogenic genomic clone, e.g. from a mouse strain such as 129/Sv (Stratagene Inc., La Jolla, CA). The targeting vector may be introduced into a suitably-derived line of embryonic stem (ES) cells by electroporation to generate ES cell lines that carry a profoundly truncated form of the *KCNQ5* gene. The targeted cell lines may then be injected into a mouse blastula stage embryo to generate chimeric founder mice. Heterozygous offspring may be interbred to homozygosity.

Animal models for over-expression may be generated by integrating one or more polynucleotide sequence of the invention into the genome according to standard techniques.

The procedures disclosed herein involving the molecular manipulation of nucleic acids are known to those skilled in the art, and are described by e.g. *Fredrick MA et al.* [Fredrick MA et al.: Short Protocols in Molecular Biology; John Wiley and Sons, 1995] and *Sambrook et al.* [Sambrook et al.: Molecular Cloning: A Laboratory Manual; 2. Ed., Cold Spring Harbor Lab.; Cold Spring Harbor, NY 1989], and in Alexandra LJ (Ed.): Gene Targeting: A practical approach; Oxford University Press (Oxford, New York, Tokyo), 1993.

### BRIEF DESCRIPTION OF THE DRAWINGS

The present invention is further illustrated by reference to the accompanying drawing, in which:

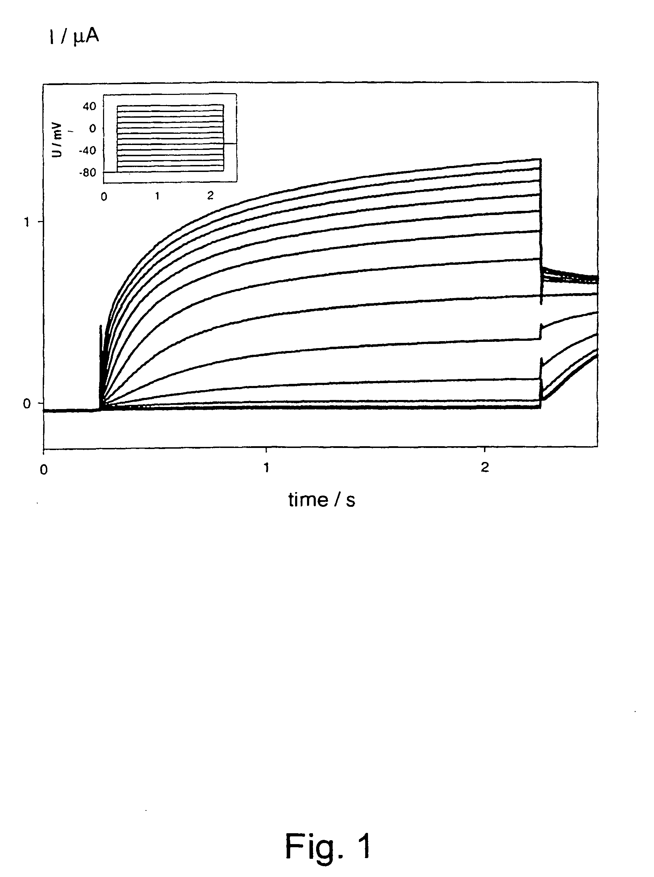

Fig. 1 shows the electrophysiological properties of KCNQ5 currents [I/µA vs. time/seconds]. Two-electrode voltage-clamp current traces from a *Xenopus* oocyte injected with KCNQ5 cRNA. Starting from a holding potential of -80 mV, cells were clamped for 2 seconds to voltages between -80 and +40 mV, in +10 mV steps, followed by a constant test pulse to -30 mV; and

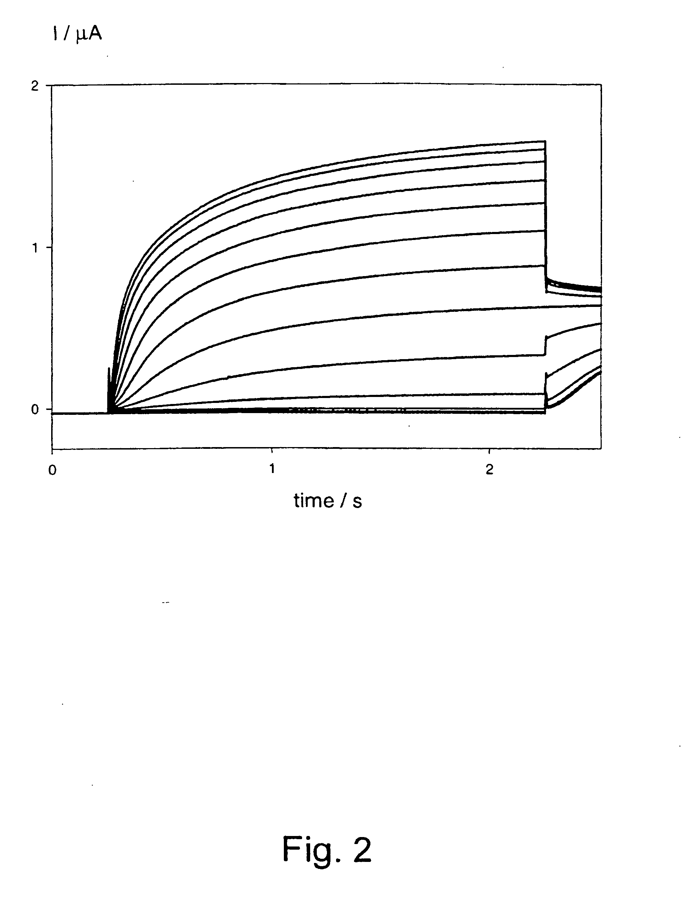

Fig. 2 shows the electrophysiological properties of currents arising from the co-expression of KCNQ5 with KCNQ3 [l/µA vs. time/seconds]. Starting from a holding potential of -80 mV cells were clamped for 2 seconds to voltages between -80 and +40 mV, in +10 mV steps, followed by a constant test pulse to -30 mV; and

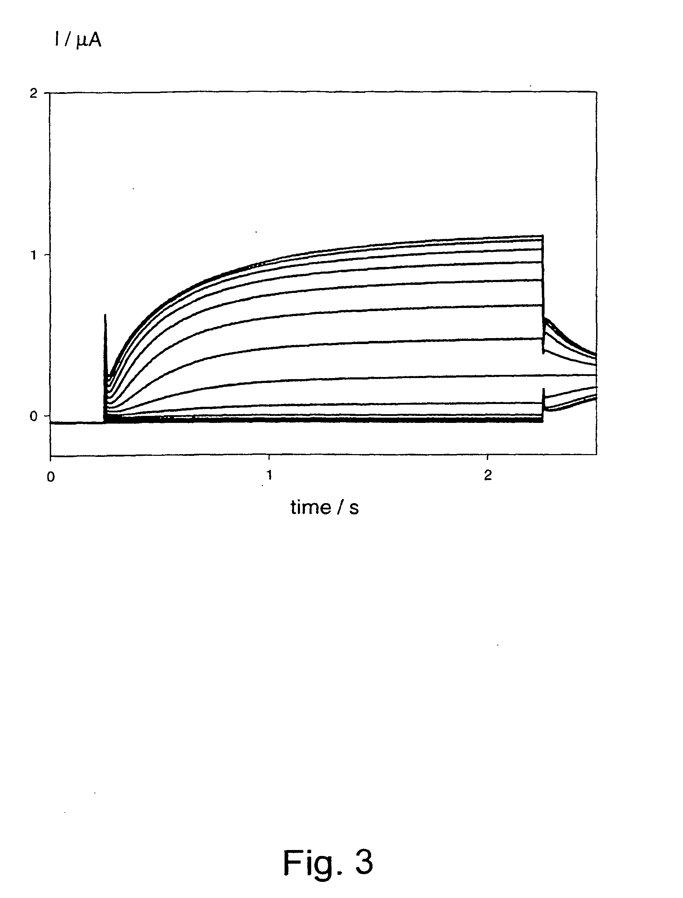

Fig. 3 shows the electrophysiological properties of currents arising from the co-expression of KCNQ5 with KCNQ4 (2B) [l/µA vs. time/seconds]. Starting from a holding potential of -80 mV cells were clamped for 2 seconds to voltages between -80 and +40 mV, in +10 mV steps, followed by a constant test pulse to -30 mV;

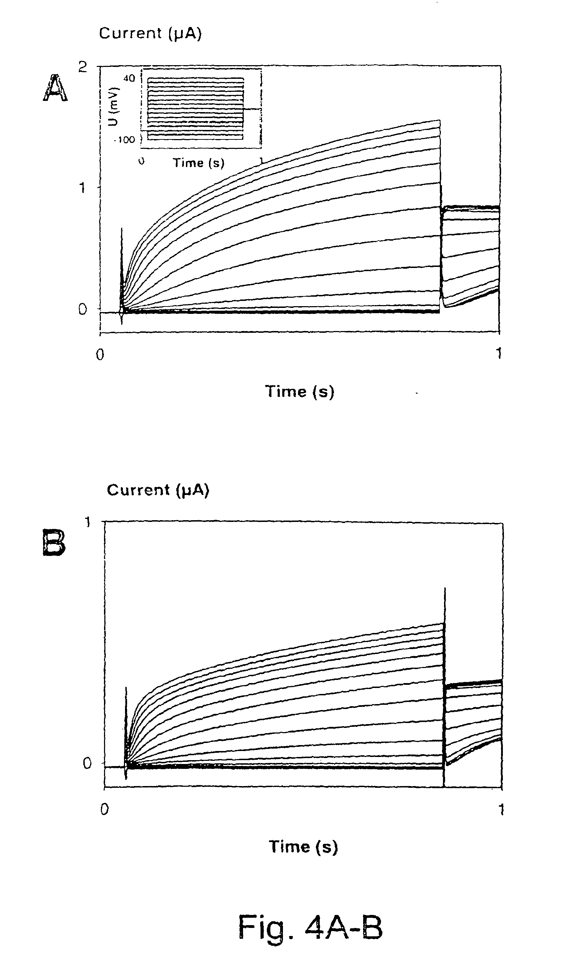

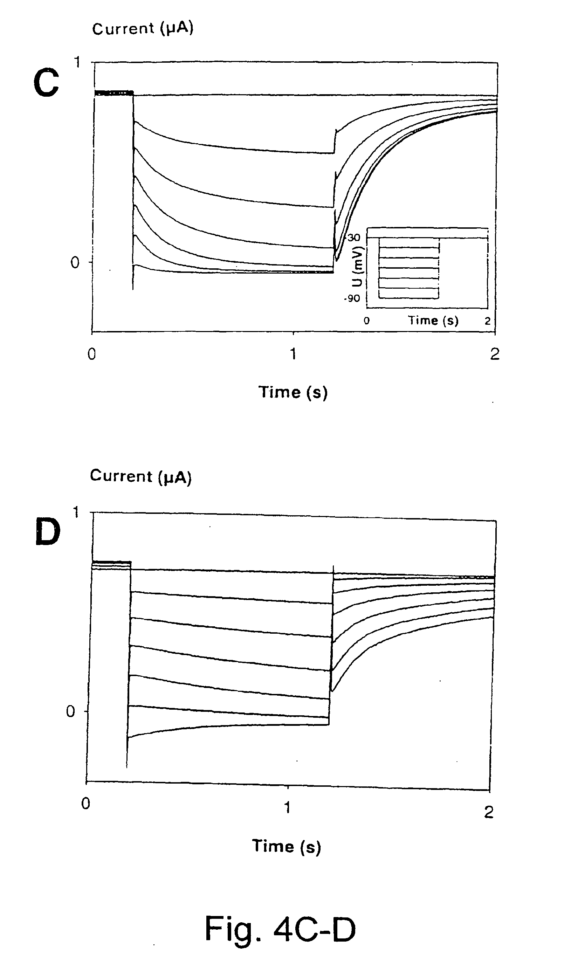

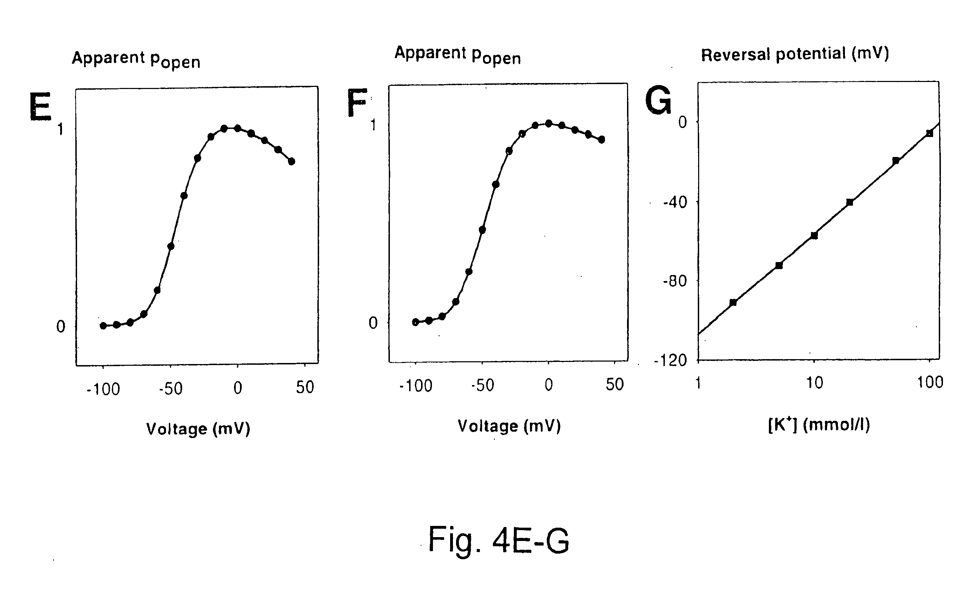

Figs. 4A-G show the electrophysiological properties of KCNQ5. Both the splice variant I (found in brain) and splice variant III (found in muscle) were expressed in *Xenopus* oocytes and examined by two-electrode voltage clamping. Both variants activate slowly upon depolarisation, but form I (4A) initially activates slower than the muscle form III (4B). Starting from a holding potential of -80 mV, the voltage was stepped for 0.8 seconds to values between -100 and + 40 mV in steps of 10 mV, followed by a voltage step to -30 mV (see inset, panel A). Channel activation by depolarization was fitted (for 2 sec. steps) by a sum of three exponential functions. For a step to +20 mV, the rate constants were ô₁ = 37. 2 ± 2.2 ms, ô₂ = 246 ± 17 ms, and ô₃ = 1112 ± 91 ms for splice variant I, while these constants were ô₁ = 24.5 ± 0.8 ms, ô₂ = 163 ± 6 ms, and ô₃ = 1690 ± 46 ms (± SEM, n=16). This difference in kinetics also results in different curves when a typical M-current protocol is used (4C, variant I; 4D, variant III). Variant I induces currents that kinetically resemble M-currents. In this protocol, the membrane voltage is clamped for 1 second to voltages between -30 and -90 mV in steps of -10 mV, from a holding potential of -30 mV. This was followed by a step to -30 mV (panel C, inset). (4E, 4F), apparent open probabilities of variants I (4E) and variant III (4F) as a function of voltage obtained from tail current analysis as described in Methods. Mean values obtained from 12 oocytes are shown. Fitting a Boltzmann equation yielded V_{½} = -46 ± 1 mV and an apparent gating charge of z = 2.8 ± 0.1 for isoform I, and V_{½} = -48 ± 1 mV and an apparent gating charge of z = 2.5 ± 0.1 for isoform III. For this fit, values at potentials more positive than +10 mV were excluded, as these are probably affected by a second (inactivation) gating process which leads to a decrease of apparent Pₒₚₑₙ at more positive potentials. (4G), ion selectivity of KCNQ5 currents (variant I). Extracellular sodium was replaced by equimolar amounts of potassium. The reversal potential is shown as a function of the potassium concentration. This yielded a slope of 51 mV/decade potassium concentration, indicating a highly selective potassium channel. Data are from 10 oocytes from 2 different batches;

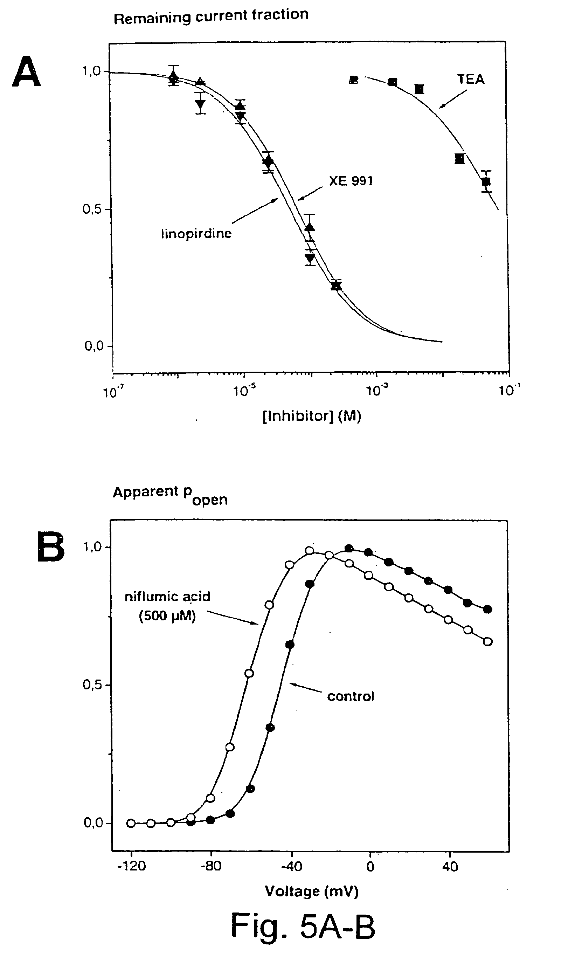

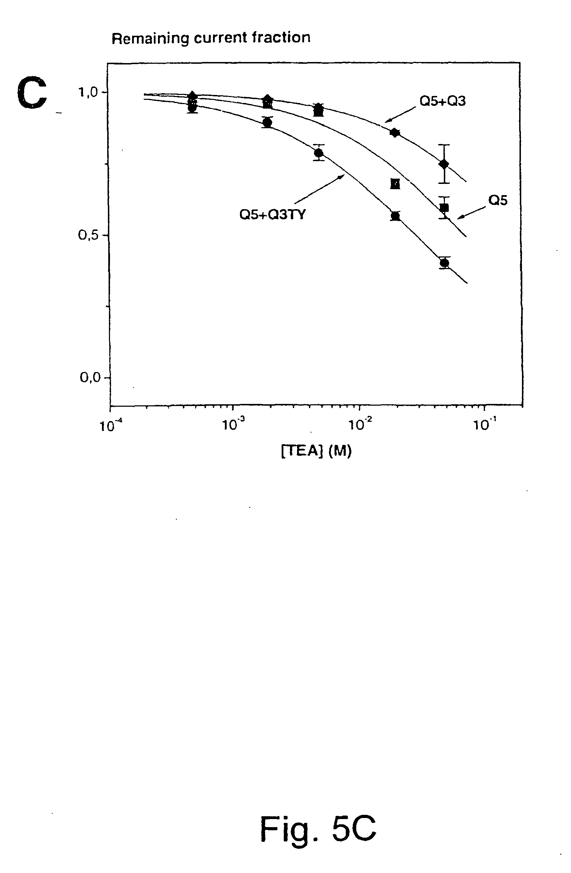

Figs. 5A-C show the pharmacology of KCNQ5 and KCNQ3/5 heteromers. (5A) Inhibition of KCNQ5 homomers by extracellular linopirdine (*down triangles*), XE991 (*up triangles*) and TEA (*squares*)*.* IC₅₀ values of 51 ± 5 µM, 65 ± 4 µM and 71 ± 17 mM, respectively, were obtained from the plotted fit curves. (5B) Niflumic acid alters the voltage dependence of the apparent pₒₚₑₙ. In the presence of 500 µM niflumic acid (*open circles*), the voltage dependence is shifted about 20 mV towards negative potentials. (5C) TEA sensitivity is altered in KCNQ3/5 heteromers. Coexpression of KCNQ5 and KCNQ3 (1:1) (*diamonds*) increased the IC₅₀ value to 200 mM, coexpression with the KCNQ3 (T323Y) mutant (*circles*) decreased the IC₅₀ to ~ 30 mM. Data points in panels A and C are the means ± SEM of 4 to 12 individual measurements. pₒₚₑₙ was determined as in Fig. 3;

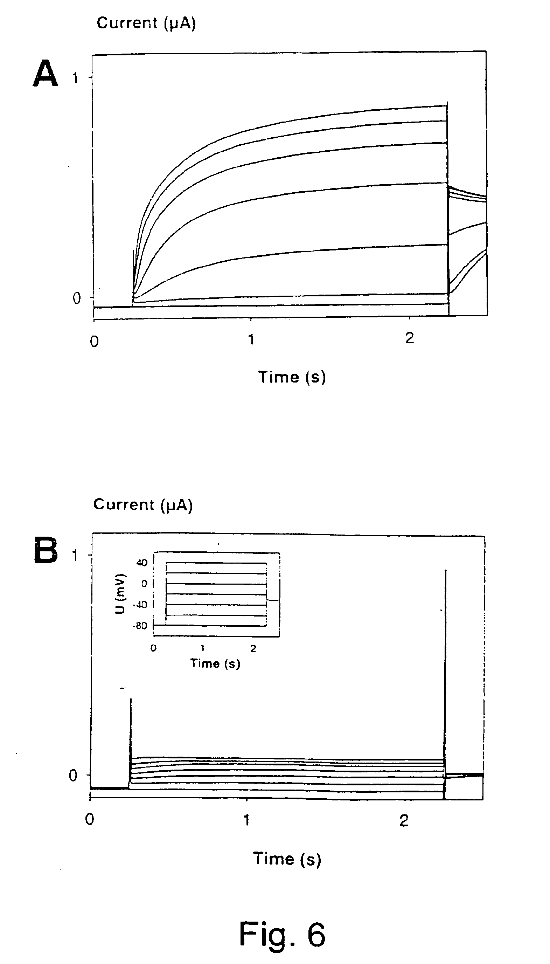

Fig. 6 shows the inhibition of KCNQ5 currents by stimulating M1 receptors which were co-expressed in *Xenopus* oocytes:
   (6A) currents before stimulating the M1 receptor;
   (6B) current observed 3 minutes after applying 10 µM muscarine. The pulse protocol is shown in the inset of panel b. No effect of 10 µM muscarine or oxotremorine methiodide was found in oocytes injected with KCNQ5 alone; and

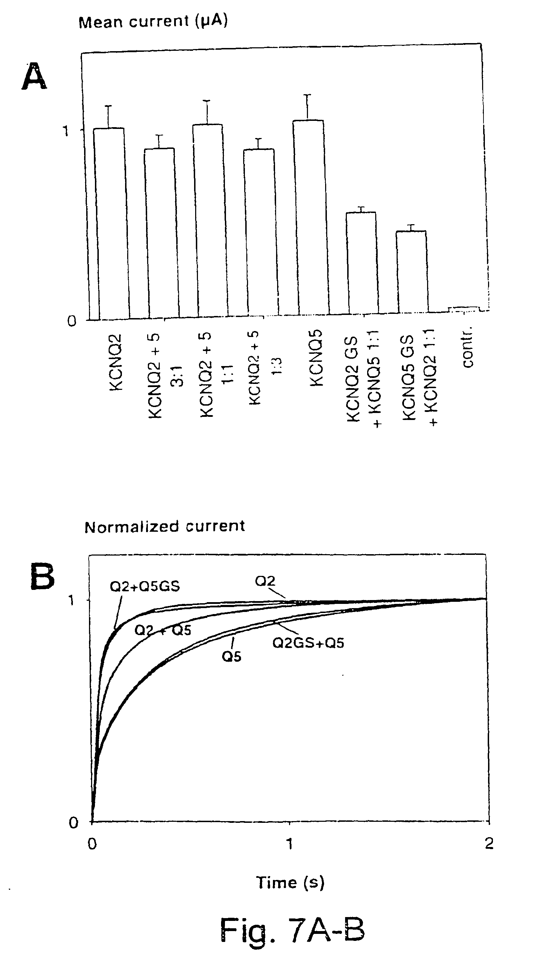

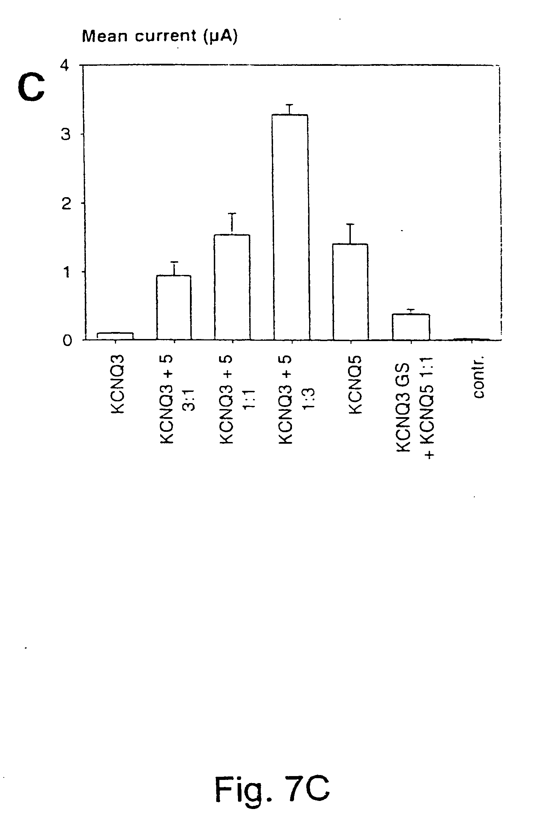

Figs. 7A-C show the interactions between KCNQ2 and KCNQ5 (7A, 7B) and KCNQ3 and KCNQ5 (7C):
   (7A) currents at the end of a two second pulse to 0 mV from a holding potential of -80 mV of oocytes injected with different combinations of KCNQ cRNAs (always 10 ng total amount of RNA). The current amplitude elicited by co-injecting KCNQ2 and KCNQ5 could be explained by a linear superposition of currents. Co-injection of KCNQ5 with the dominant negative mutant KCNQ2(G279S) or of KCNQ2 with the equivalent mutant KCNQ5(G278S) lead to a roughly 50% reduction in current amplitude, which is consistent with a lack of interaction since only 50% of WT cRNA was injected;
   (7B) normalized current traces of experiments used for panel (A). Currents elicited by the depolarizing pulse to 0 mV are shown. KCNQ2 (labeled Q2) activates faster than KCNQ5 (Q5), and the co-expression of both yielded currents that may be explained by a linear superposition. Co-injecting KCNQ2 with the dominant negative (and otherwise non-functional) mutant KCNQ5(G278S) yielded currents that were kinetically similar to KCNQ2, and currents from a KCNQ5/KCNQ2(G279S) co-injection resembled KCNQ5 currents;
   (7C), interactions between KCNQ3 and KCNQ5 measured as in panel (a). KCNQ3 yields only very small currents in Xenopus oocytes. Currents were enlarged when KCNQ5 was co-expressed with small amounts of KCNQ3, and reduced when co-expressed with larger amounts. The dominant negative mutant KCNQ3(G318S) decreased currents significantly below 50% of WT KCNQ5 currents that would be expected from the injected 50% of WT KCNQ5 cRNA in this experiment.

### EXAMPLES

The invention is further illustrated with reference to the following examples which are not intended to be in any way limiting to the scope of the invention as claimed.

### Example 1

### Cloning and Characterisation of KCNQ5 cDNA

Using a near full-length KCNQ3 potassium channel cDNA as a probe, a human thalamus cDNA λGT11 phage library (Clontech, #HL5009b) was screened, and a partial cDNA clone encoding a protein fragment homologous to KCNQ potassium channels was isolated. It was distinct from the known members KCNQ1 (KvLQT1), KCNQ2, KCNQ3 and KCNQ4. We named the novel gene *KCNQ5.* Overlapping cDNA's containing the entire open reading frame were obtained by re-screening the cDNA library and by extending the 5' end in RACE (rapid amplification of cDNA ends) experiments using a Marathon kit (Clontech) with human brain cDNA. A complete cDNA was assembled and cloned into the oocyte expression vector PTLN [Lorenz C, Pusch M & Jentsch T J: Heteromultimeric CLC chloride channels with novel properties; Proc. Natl. Acad. Sci. USA 1996 93 13362-13366].

The cDNA encodes a polypeptide of 897 amino acids with a predicted mass of 99 kDa (SEQ ID NO: 2). Its overall amino-acid identity to KCNQ1, KCNQ2, KCNQ3 and KCNQ4 is 43%, 58%, 54%, and 61 % respectively. Together with these proteins, it forms a distinct branch of the superfamily of voltage-gated potassium channels. As a typical member of this gene family, KCNQ5 has 6 predicted transmembrane domains and a P-loop between transmembrane domains S5 and S6. In potassium channels, which are tetramers of identical or homologous subunits, four of these highly conserved P-loops combine to form the ion-selective pore. As other KCNQ channels, KCNQ5 has a long predicted cytoplasmic carboxy terminus that accounts for about half of the protein. A conserved region present in the carboxy termini of KCNQ1, -2, -3 and -4 is also present in KCNQ5.

The sequence of KCNQ5 predicts several potential sites for phosphorylation by protein kinase C and one for protein kinase A. In contrast to KCNQ1 and KCNQ2, however, it lacks an amino terminal consensus site for cAMP-dependent phosphorylation.

A human multiple tissue Northern blot (Clontech, #7760-1) was probed with a cDNA fragment of KCNQ5. The fragment was labelled with ³²P using the Rediprime labelling kit (Amersham). Hybridisation was performed in ExpressHyb solution according to the instructions of the manufacturer (Clontech). The filter was then exposed to Kodak BioMax film for 4 days.

Northern analysis of *KCNQ5* expression in human tissues revealed a band of ≈9 kb in brain.

### Example 2

### Functional expression of KCNQ5 potassium channel subunits

KCNQ5 was expressed in *Xenopus* oocytes and its activity was investigated by two-electrode voltage clamping.

After linearization of the *KCNQ5*-containing pTLN vector with Hpal, capped cRNA was transcribed *in vitro* using the mMessage mMachine cRNA synthesis kit (Ambion). Usually 5 - 15 ng of cRNA were injected into *Xenopus* oocytes previously isolated by manual defolliculation and short collagenase treatment. In co-expression experiments cRNAs were injected at a 1:1 ratio. Oocytes were kept at 17°C in modified Barth's solution (90 mM NaCl, 1 mM KCI, 0.41 mM CaCl₂, 0.33 mM Ca(NO₃)₂, 0.82 mM MgSO₄, 10 mM HEPES, 100 U penicillin-100 µg streptomycin/ml, pH 7.6).

Standard two-electrode voltage-clamp measurements were performed at room temperature 2-4 days after injection using a Turbotec 05 amplifier (npi instruments, Tamm, Germany) and pClamp 5.5 software (Axon Instruments). Currents were usually recorded in ND98 solution (see Table 2). Linopirdine (RBI, Natick, MA) was prepared as a 100 mM stock solution in DMSO and added to a final concentration of 200 µM to ND98.

**Table 2**

| **Solution contents (Concentrations in mM)** | | | | |
|---|---|---|---|---|
| ND98 | ND 100 | KD100 | Rb100 | Cs100 |
| 98 NaCl | 100 NaCl | 100 KCI | 100 RbCl | 100 CsCl |
| 2 KCI | | | | |
| 0.2 CaCl₂ | 0.2 CaCl₂ | 0.2 CaCl₂ | 0.2 CaCl₂ | 0.2 CaCl₂ |
| 2.8 MgCl₂ | 2.8 MgCl₂ | 2.8 MgCl₂ | 2.8 MgCl₂ | 2.8 MgCl₂ |
| 5 mM HEPES, pH 7.4 | | | | |

To determine the voltage dependence of apparent open probability, oocytes were clamped for 2 seconds to values between -80 mV to +40 mV, in 10 mV steps, followed by a constant -30 mV test pulse. Tail currents extrapolated to t=0 were obtained from a mono-exponential fit, normalised to the value at 0 mV and used for the analysis of apparent pₒₚₑₙ. Data analysis used PClamp6 and Microcal Origin 5.0.

Similar to KCNQ1, KCNQ2, KCNQ3, and KCNQ4 also KCNQ5 yielded currents that activated upon depolarisation (Fig. 1). Compared to KCNQ1, KCNQ2 and KCNQ2/3 channels, however, current activation was slower and occurred with a time constant in the order of 600 ms at +20mV (KCNQ2/KCNQ3 channels have a corresponding time constant of ≈300 ms). Deactivation of currents at physiological resting potentials (≈-30mV) was considerably faster (Fig. 1). Similar to KCNQ2, macroscopic currents often showed some inward rectification at positive potentials. KCNQ5 currents were inhibited by more than 80% by 5 mM Ba⁺⁺.

KCNQ1 assembles with minK (also known as KCNE1 or IsK) to form channels that yield larger currents and activate much slower. We therefore tested by co-expression whether minK affects KCNQ5 as well. At concentrations (1ng minK cRNA per oocyte) leading to drastic changes in KCNQ1 currents in parallel experiments, there was just a slight change in KCNQ5 currents.

Different KCNQ subunits can form heteromeric channels. Co-expression of KCNQ2 with KCNQ3, but not with KCNQ1, gave currents that were about tenfold larger than those from homomeric channels. Since also KCNQ2, KCNQ3 and KCNQ4 are expressed in the brain, we investigated whether these proteins interact functionally. Oocytes co-injected (at the same total cRNA concentration) with KCNQ2 and KCNQ5 cRNAs yielded currents that seemed not different from a linear superposition of currents from the respective homomeric channels.

By contrast, co-expression of KCNQ3 with KCNQ5 yielded currents that were significantly larger than could be explained by a superposition of currents from the respective homomeric channels (Figs. 2A). Further, co-expression of KCNQ5 and KCNQ4 decreased currents when compared to homomeric channels (Fig. 2B).

Linopirdine, a potent and rather specific inhibitor for M-currents, nearly completely inhibits KCNQ2/KCNQ3 channels at a concentration of 200 µM.

This concentration of Linopirdine inhibited KCNQ5 by about 80%.

### SEQUENCE LISTING

(2) INFORMATION FOR SEQ ID NO: 1:
   (i) SEQUENCE CHARACTERISTICS:
      (A) LENGTH: 3137 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (vi) ORIGINAL SOURCE:
      (A) ORGANISM: Homo sapiens
   (vii) IMMEDIATE SOURCE:
      (B) CLONE: KCNQ5
   (ix) FEATURE:
      (A) NAME/KEY: CDS
      (B) LOCATION: 1..2694
   (x) SEQUENCE DESCRIPTION: SEQ ID NO: 1:
2) INFORMATION FOR SEQ ID NO: 2:
   (i) SEQUENCE CHARACTERISTICS:
      (A) LENGTH: 897 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Lys Asp Val Glu Ser Gly Arg Gly Arg Val Leu Leu Asn Ser Ala
    1               5                  10                  15
   Ala Ala Arg Gly Asp Gly Leu Leu Leu Leu Gly Thr Arg Ala Ala Thr
               20                  25                  30
   Leu Gly Gly Gly Gly Gly Gly Leu Arg Glu Ser Arg Arg Gly Lys Gln
           35                  40                  45
   Gly Ala Arg Met Ser Leu Leu Gly Lys Pro Leu Ser Tyr Thr Ser Ser
       50                  55                  60
   Gln Ser Cys Arg Arg Asn Val Lys Tyr Arg Arg Val Gln Asn Tyr Leu
   65                  70                  75                  80
   Tyr Asn Val Leu Glu Arg Pro Arg Gly Trp Ala Phe Ile Tyr His Ala
                   85                  90                  95
   Phe Val Phe Leu Leu Val Phe Gly Cys Leu Ile Leu Ser Val Phe Ser
               100                 105                 110
   Thr Ile Pro Glu His Thr Lys Leu Ala Ser Ser Cys Leu Leu Ile Leu
           115                 120                 125
   Glu Phe Val Met Ile Val Val Phe Gly Leu Glu Phe Ile Ile Arg Ile
       130                 135                 140
   Trp Ser Ala Gly Cys Cys Cys Arg Tyr Arg Gly Trp Gln Gly Arg Leu
   145                 150                 155                 160
   Arg Phe Ala Arg Lys Pro Phe Cys Val Ile Asp Thr Ile Val Leu Ile
                   165                 170                 175
   Ala Ser Ile Ala Val Val Ser Ala Lys Thr Gln Gly Asn Ile Phe Ala
               180                 185                 190
   Thr Ser Ala Leu Arg Ser Leu Arg Phe Leu Gln Ile Leu Arg Met Val
           195                 200                 205
   Arg Met Asp Arg Arg Gly Gly Thr Trp Lys Leu Leu Gly Ser Val Val
       210                 215                 220
   Tyr Ala His Ser Lys Glu Leu Ile Thr Ala Trp Tyr Ile Gly Phe Leu
   225                 230                 235                 240
   Val Leu Ile Phe Ser Ser Phe Leu Val Tyr Leu Val Glu Lys Asp Ala
                   245                 250                 255
   Asn Lys Glu Phe Ser Thr Tyr Ala Asp Ala Leu Trp Trp Gly Thr Ile
               260                 265                 270
   Thr Leu Thr Thr Ile Gly Tyr Gly Asp Lys Thr Pro Leu Thr Trp Leu
           275                 280                 285
   Gly Arg Leu Leu Ser Ala Gly Phe Ala Leu Leu Gly Ile Ser Phe Phe
       290                 295                 300
   Ala Leu Pro Ala Gly Ile Leu Gly Ser Gly Phe Ala Leu Lys Val Gln
   305                 310                 315                 320
   Glu Gln His Arg Gln Lys His Phe Glu Lys Arg Arg Asn Pro Ala Ala
                   325                 330                 335
   Asn Leu Ile Gln Cys Val Trp Arg Ser Tyr Ala Ala Asp Glu Lys Ser
               340                 345                 350
   Val Ser Ile Ala Thr Trp Lys Pro His Leu Lys Ala Leu His Thr Cys
           355                 360                 365
   Ser Pro Thr Lys Lys Glu Gln Gly Glu Ala Ser Ser Ser Gln Lys Leu
       370                 375                 380
   Ser Phe Lys Glu Arg Val Arg Met Ala Ser Pro Arg Gly Gln Ser Ile
   385                 390                 395                 400
   Lys Ser Arg Gln Ala Ser Val Gly Asp Arg Arg Ser Pro Ser Thr Asp
                   405                 410                 415
   Ile Thr Ala Glu Gly Ser Pro Thr Lys Val Gln Lys Ser Trp Ser Phe
               420                 425                 430
   Asn Asp Arg Thr Arg Phe Arg Pro Ser Leu Arg Leu Lys Ser Ser Gln
           435                 440                 445
   Pro Lys Pro Val Ile Asp Ala Asp Thr Ala Leu Gly Thr Asp Asp Val
       450                 455                 460
   Tyr Asp Glu Lys Gly Cys Gln Cys Asp Val Ser Val Glu Asp Leu Thr
   465                 470                 475                 480
   Pro Pro Leu Lys Thr Val Ile Arg Ala Ile Arg Ile Met Lys Phe His
                   485                 490                 495
   Val Ala Lys Arg Lys Phe Lys Glu Thr Leu Arg Pro Tyr Asp Val Lys
               500                 505                 510
   Asp Val Ile Glu Gln Tyr Ser Ala Gly His Leu Asp Met Leu Cys Arg
           515                 520                 525
   Ile Lys Ser Leu Gln Thr Arg Val Asp Gln Ile Leu Gly Lys Gly Gln
       530                 535                 540
   Ile Thr Ser Asp Lys Lys Ser Arg Glu Lys Ile Thr Ala Glu His Glu
   545                 550                 555                 560
   Thr Thr Asp Asp Leu Ser Met Leu Gly Arg Val Val Lys Val Glu Lys
                   565                 570                 575
   Gln Val Gln Ser Ile Glu Ser Lys Leu Asp Cys Leu Leu Asp Ile Tyr
               580                 585                 590
   Gln Gln Val Leu Arg Lys Gly Ser Ala Ser Ala Leu Ala Leu Ala Ser
           595                 600                 605
   Phe Gln Ile Pro Pro Phe Glu Cys Glu Gln Thr Ser Asp Tyr Gln Ser
       610                 615                 620
   Pro Val Asp Ser Lys Asp Leu Ser Gly Ser Ala Gln Asn Ser Gly Cys
   625                 630                 635                 640
   Leu Ser Arg Ser Thr Ser Ala Asn Ile Ser Arg Gly Leu Gln Phe Ile
                   645                 650                 655
   Leu Thr Pro Asn Glu Phe Ser Ala Gln Thr Phe Tyr Ala Leu Ser Pro
               660                 665                 670
   Thr Met His Ser Gln Ala Thr Gln Val Pro Ile Ser Gln Ser Asp Gly
           675                 680                 685
   Ser Ala Val Ala Ala Thr Asn Thr Ile Ala Asn Gln Ile Asn Thr Ala
       690                 695                 700
   Pro Lys Pro Ala Ala Pro Thr Thr Leu Gln Ile Pro Pro Pro Leu Pro
   705                 710                 715                 720
   Ala Ile Lys His Leu Pro Arg Pro Glu Thr Leu His Pro Asn Pro Ala
                   725                 730                 735
   Gly Leu Gln Glu Ser Ile Ser Asp Val Thr Thr Cys Leu Val Ala Ser
               740                 745                 750
   Lys Glu Asn Val Gln Val Ala Gln Ser Asn Leu Thr Lys Asp Arg Ser
           755                 760                 765
   Met Arg Lys Ser Phe Asp Met Gly Gly Glu Thr Leu Leu Ser Val Cys
       770                 775                 780
   Pro Met Val Pro Lys Asp Leu Gly Lys Ser Leu Ser Val Gln Asn Leu
   785                 790                 795                 800
   Ile Arg Ser Thr Glu Glu Leu Asn Ile Gln Leu Ser Gly Ser Glu Ser
                   805                 810                 815
   Ser Gly Ser Arg Gly Ser Gln Asp Phe Tyr Pro Lys Trp Arg Glu Ser
               820                 825                 830
   Lys Leu Phe Ile Thr Asp Glu Glu Val Gly Pro Glu Glu Thr Glu Thr
           835                 840                 845
   Asp Thr Phe Asp Ala Ala Pro Gln Pro Ala Arg Glu Ala Ala Phe Ala
       850                 855                 860
   Ser Asp Ser Leu Arg Thr Gly Arg Ser Arg Ser Ser Gln Ser Ile Cys
   865                 870                 875                 880
   Lys Ala Gly Glu Ser Thr Asp Ala Leu Ser Leu Pro His Val Lys Leu
                   885                 890                 895
   Lys

### SEQUENCE LISTING

<110> NeuroSearch A/S
<120> NOVEL POTASSIUM CHANNELS AND GENES ENCODING THESE
   POTASSIUM CHANNELS
<130> 136-204-WO
<140>
   <141>
<160> 2
<170> PatentIn Ver. 2.1
<210> 1
   <211> 3137
   <212> DNA
   <213> Homo sapiens
<220>
   <221> CDS
   <222> (1)..(2691)
<400> 1
   atg aag gat gtg gag tcg ggc cgg ggc agg gtg ctg ctg aac tcg gca      48
   Met Lys Asp Val Glu Ser Gly Arg Gly Arg Val Leu Leu Asn Ser Ala
    1               5                  10                  15
   gcc gcc agg ggc gac ggc ctg cta ctg ctg ggc acc cgc gcg gcc acg      96
   Ala Ala Arg Gly Asp Gly Leu Leu Leu Leu Gly Thr Arg Ala Ala Thr
               20                  25                  30
   ctc ggt ggc ggc ggc ggt ggc ctg agg gag agc cgc cgg ggc aag cag     144
   Leu Gly Gly Gly Gly Gly Gly Leu Arg Glu Ser Arg Arg Gly Lys Gln
           35                  40                  45
   ggg gcc cgg atg agc ctg ctg ggg aag ccg ctc tct tac acg agt agc     192
   Gly Ala Arg Met Ser Leu Leu Gly Lys Pro Leu Ser Tyr Thr Ser Ser
       50                  55                  60
   cag agc tgc cgg cgc aac gtc aag tac cgg cgg gtg cag aac tac ctg     240
   Gln Ser Cys Arg Arg Asn Val Lys Tyr Arg Arg Val Gln Asn Tyr Leu
   65                  70                  75                  80
   tac aac gtg ctg gag aga ccc cgc ggc tgg gcg ttc atc tac cac gct     288
   Tyr Asn Val Leu Glu Arg Pro Arg Gly Trp Ala Phe Ile Tyr His Ala
                   85                  90                  95
   ttc gtt ttt ctc ctt gtc ttt ggt tgc ttg att ttg tca gtg ttt tct     336
   Phe Val Phe Leu Leu Val Phe Gly Cys Leu Ile Leu Ser Val Phe Ser
               100                 105                 110
   acc atc cct gag cac aca aaa ttg gcc tca agt tgc ctc ttg atc ctg     384
   Thr Ile Pro Glu His Thr Lys Leu Ala Ser Ser Cys Leu Leu Ile Leu
           115                 120                 125
   gag ttc gtg atg att gtc gtc ttt ggt ttg gag ttc atc att cga atc     432
   Glu Phe Val Met Ile Val Val Phe Gly Leu Glu Phe Ile Ile Arg Ile
       130                 135                 140
   tgg tct gcg ggt tgc tgt tgt cga tat aga gga tgg caa gga aga ctg     480
   Trp Ser Ala Gly Cys Cys Cys Arg Tyr Arg Gly Trp Gln Gly Arg Leu
   145                 150                 155                 160
   agg ttt gct cga aag ccc ttc tgt gtt ata gat acc att gtt ctt atc      528
   Arg Phe Ala Arg Lys Pro Phe Cys Val Ile Asp Thr Ile Val Leu Ile
                   165                 170                 175
   gct tca ata gca gtt gtt tct gca aaa act cag ggt aat att ttt gcc      576
   Ala Ser Ile Ala Val Val Ser Ala Lys Thr Gln Gly Asn Ile Phe Ala
               180                 185                 190
   acg tct gca ctc aga agt ctc cgt ttc cta cag atc ctc cgc atg gtg      624
   Thr Ser Ala Leu Arg Ser Leu Arg Phe Leu Gln Ile Leu Arg Met Val
           195                 200                 205
   cgc atg gac cga agg gga ggc act tgg aaa tta ctg ggt tca gtg gtt      672
   Arg Met Asp Arg Arg Gly Gly Thr Trp Lys Leu Leu Gly Ser Val Val
       210                 215                 220
   tat gct cac agc aag gaa tta atc aca gct tgg tac ata gga ttt ttg      720
   Tyr Ala His Ser Lys Glu Leu Ile Thr Ala Trp Tyr Ile Gly Phe Leu
   225                 230                 235                 240
   gtt ctt att ttt tcg tct ttc ctt gtc tat ctg gtg gaa aag gat gcc      768
   Val Leu Ile Phe Ser Ser Phe Leu Val Tyr Leu Val Glu Lys Asp Ala
                   245                 250                 255
   aat aaa gag ttt tct aca tat gca gat gct ctc tgg tgg ggc aca att      816
   Asn Lys Glu Phe Ser Thr Tyr Ala Asp Ala Leu Trp Trp Gly Thr Ile
               260                 265                 270
   aca ttg aca act att ggc tat gga gac aaa act ccc cta act tgg ctg      864
   Thr Leu Thr Thr Ile Gly Tyr Gly Asp Lys Thr Pro Leu Thr Trp Leu
           275                 280                 285
   gga aga ttg ctt tct gca ggc ttt gca ctc ctt ggc att tct ttc ttt      912
   Gly Arg Leu Leu Ser Ala Gly Phe Ala Leu Leu Gly Ile Ser Phe Phe
       290                 295                 300
   gca ctt cct gcc ggc att ctt ggc tca ggt ttt gca tta aaa gta caa      960
   Ala Leu Pro Ala Gly Ile Leu Gly Ser Gly Phe Ala Leu Lys Val Gln
   305                 310                 315                 320
   gaa caa cac cgc cag aaa cac ttt gag aaa aga agg aac cca gct gcc     1008
   Glu Gln His Arg Gln Lys His Phe Glu Lys Arg Arg Asn Pro Ala Ala
                   325                 330                 335
   aac ctc att cag tgt gtt tgg cgt agt tac gca gct gat gag aaa tct     1056
   Asn Leu Ile Gln Cys Val Trp Arg Ser Tyr Ala Ala Asp Glu Lys Ser
               340                 345                 350
   gtt tcc att gca acc tgg aag cca cac ttg aag gcc ttg cac acc tgc     1104
   Val Ser Ile Ala Thr Trp Lys Pro His Leu Lys Ala Leu His Thr Cys
           355                 360                 365
   agc cct acc aag aaa gaa caa ggg gaa gca tca agc agt cag aag cta     1152
   Ser Pro Thr Lys Lys Glu Gln Gly Glu Ala Ser Ser Ser Gln Lys Leu
       370                 375                 380
   agt ttt aag gag cga gtg cgc atg gct agc ccc agg ggc cag agt att     1200
   Ser Phe Lys Glu Arg Val Arg Met Ala Ser Pro Arg Gly Gln Ser Ile
   385                 390                 395                 400
   aag agc cga caa gcc tca gta ggt gac agg agg tcc cca agc acc gac     1248
   Lys Ser Arg Gln Ala Ser Val Gly Asp Arg Arg Ser Pro Ser Thr Asp
                   405                 410                 415
   atc aca gcc gag ggc agt ccc acc aaa gtg cag aag agc tgg agc ttc     1296
   Ile Thr Ala Glu Gly Ser Pro Thr Lys Val Gln Lys Ser Trp Ser Phe
               420                 425                 430
   aac gac cga acc cgc ttc cgg ccc tcg ctg cgc ctc aaa agt tct cag 1344
   Asn Asp Arg Thr Arg Phe Arg Pro Ser Leu Arg Leu Lys Ser Ser Gln
   435 440 445
   cca aaa cca gtg ata gat gct gac aca gcc ctt ggc act gat gat gta 1392
   Pro Lys Pro Val Ile Asp Ala Asp Thr Ala Leu Gly Thr Asp Asp Val
   450 455 460
   tat gat gaa aaa gga tgc cag tgt gat gta tca gtg gaa gac ctc acc 1440
   Tyr Asp Glu Lys Gly Cys Gln Cys Asp Val Ser Val Glu Asp Leu Thr
   465 470 475 480
   cca cca ctt aaa act gtc att cga gct atc aga att atg aaa ttt cat 1488
   Pro Pro Leu Lys Thr Val Ile Arg Ala Ile Arg Ile Met Lys Phe His
   485 490 495
   gtt gca aaa cgg aag ttt aag gaa aca tta cgt cca tat gat gta aaa 1536
   Val Ala Lys Arg Lys Phe Lys Glu Thr Leu Arg Pro Tyr Asp Val Lys
   500 505 510
   gat gtc att gaa caa tat tct gct ggt cat ctg gac atg ttg tgt aga 1584
   Asp Val Ile Glu Gln Tyr Ser Ala Gly His Leu Asp Met Leu Cys Arg
   515 520 525
   att aaa agc ctt caa aca cgt gtt gat caa att ctt gga aaa ggg caa 1632
   Ile Lys Ser Leu Gln Thr Arg Val Asp Gln Ile Leu Gly Lys Gly Gln
   530 535 540
   atc aca tca gat aag aag agc cga gag aaa ata aca gca gaa cat gag 1680
   Ile Thr Ser Asp Lys Lys Ser Arg Glu Lys Ile Thr Ala Glu His Glu
   545 550 555 560
   acc aca gac gat ctc agt atg ctc ggt cgg gtg gtc aag gtt gaa aaa 1728
   Thr Thr Asp Asp Leu Ser Met Leu Gly Arg Val Val Lys Val Glu Lys
   565 570 575
   cag gta cag tcc ata gaa tcc aag ctg gac tgc cta cta gac atc tat 1776
   Gln Val Gln Ser Ile Glu Ser Lys Leu Asp Cys Leu Leu Asp Ile Tyr
   580 585 590
   caa cag gtc ctt cgg aaa ggc tct gcc tca gcc ctc gct ttg gct tca 1824
   Gln Gln Val Leu Arg Lys Gly Ser Ala Ser Ala Leu Ala Leu Ala Ser
   595 600 605
   ttc cag atc cca cct ttt gaa tgt gaa cag aca tct gac tat caa agc 1872
   Phe Gln Ile Pro Pro Phe Glu Cys Glu Gln Thr Ser Asp Tyr Gln Ser
   610 615 620
   cct gtg gat agc aaa gat ctt tcg ggt tcc gca caa aac agt ggc tgc 1920
   Pro Val Asp Ser Lys Asp Leu Ser Gly Ser Ala Gln Asn Ser Gly Cys
   625 630 635 640
   tta tcc aga tca act agt gcc aac atc tcg aga ggc ctg cag ttc att 1968
   Leu Ser Arg Ser Thr Ser Ala Asn Ile Ser Arg Gly Leu Gln Phe Ile
   645 650 655
   ctg acg cca aat gag ttc agt gcc cag act ttc tac gcg ctt agc cct 2016
   Leu Thr Pro Asn Glu Phe Ser Ala Gln Thr Phe Tyr Ala Leu Ser Pro
   660 665 670
   act atg cac agt caa gca aca cag gtg cca att agt caa agc gat ggc 2064
   Thr Met His Ser Gln Ala Thr Gln Val Pro Ile Ser Gln Ser Asp Gly
   675 680 685
   tca gca gtg gca gcc acc aac acc att gca aac caa ata aat acg gca 2112
   Ser Ala Val Ala Ala Thr Asn Thr Ile Ala Asn Gln Ile Asn Thr Ala
   690 695 700
   ccc aag cca gca gcc cca aca act tta cag atc cca cct cct ctc cca    2160
   Pro Lys Pro Ala Ala Pro Thr Thr Leu Gln Ile Pro Pro Pro Leu Pro
   705                 710                 715                 720
   gcc atc aag cat ctg ccc agg cca gaa act ctg cac cct aac cct gca    2208
   Ala Ile Lys His Leu Pro Arg Pro Glu Thr Leu His Pro Asn Pro Ala
                   725                 730                 735
   ggc tta cag gaa agc att tct gac gtc acc acc tgc ctt gtt gcc tcc    2256
   Gly Leu Gln Glu Ser Ile Ser Asp Val Thr Thr Cys Leu Val Ala Ser
               740                 745                 750
   aag gaa aat gtt cag gtt gca cag tca aat ctc acc aag gac cgt tct    2304
   Lys Glu Asn Val Gln Val Ala Gln Ser Asn Leu Thr Lys Asp Arg Ser
           755                 760                 765
   atg agg aaa agc ttt gac atg gga gga gaa act ctg ttg tct gtc tgt    2352
   Met Arg Lys Ser Phe Asp Met Gly Gly Glu Thr Leu Leu Ser Val Cys
       770                 775                 780
   ccc atg gtg ccg aag gac ttg ggc aaa tct ttg tct gtg caa aac ctg    2400
   Pro Met Val Pro Lys Asp Leu Gly Lys Ser Leu Ser Val Gln Asn Leu
   785                 790                 795                 800
   atc agg tcg acc gag gaa ctg aat ata caa ctt tca ggg agt gag tca    2448
   Ile Arg Ser Thr Glu Glu Leu Asn Ile Gln Leu Ser Gly Ser Glu Ser
                   805                 810                 815
   agt ggc tcc aga ggc agc caa gat ttt tac ccc aaa tgg agg gaa tcc    2496
   Ser Gly Ser Arg Gly Ser Gln Asp Phe Tyr Pro Lys Trp Arg Glu Ser
               820                 825                 830
   aaa ttg ttt ata act gat gaa gag gtg ggt ccc gaa gag aca gag aca    2544
   Lys Leu Phe Ile Thr Asp Glu Glu Val Gly Pro Glu Glu Thr Glu Thr
           835                 840                 845
   gac act ttt gat gcc gca ccg cag cct gcc agg gaa gct gcc ttt gca    2592
   Asp Thr Phe Asp Ala Ala Pro Gln Pro Ala Arg Glu Ala Ala Phe Ala
       850                 855                 860
   tca gac tct cta agg act gga agg tca cga tca tct cag agc att tgt    2640
   Ser Asp Ser Leu Arg Thr Gly Arg Ser Arg Ser Ser Gln Ser Ile Cys
   865                 870                 875                 880
   aag gca gga gaa agt aca gat gcc ctc agc ttg cct cat gtc aaa ctg    2688
   Lys Ala Gly Glu Ser Thr Asp Ala Leu Ser Leu Pro His Val Lys Leu
                   885                 890                 895
   aaa taagttcttc attttctttc caggcatagc agttctttag ccatacatat         2741
   Lys
   cattgcatga actatttcga aagcccttct aaaaagttga aattgcaaga atcgggaaga  2801
   acatgaaagg cagtttataa gcccgttacc ttttaattgc atgaaaatgc atgtttaggg  2861
   atggctaaaa ttccaaggtg catcgacatt aacccactca tttagtaatg taccttgagt  2921
   taaaaagcct gagaaaccaa acacagctaa tgctatgggg tgtatgaata tgtcaagttt  2981
   aggtcattta gaagatttga cactgtattt tgaaattatg ggagtaaaca ccttcaaatt  3041
   tcaggcattt ctgctttgtg actaaataca aactacattt tcaagattag gccataatgt  3101
   atatttaaac acaatggcta tcaacagctg ctaata                            3137
<210> 2
   <211> 897
   <212> PRT
   <213> Homo sapiens
<400> 2

## Claims

1. An isolated polynucleotide having a nucleic acid sequence which is capable of hybridising under at least medium/high stringency conditions with the polynucleotide sequence presented as SEQ ID NO: 1, or its complementary strand, wherein said hybridization occurs in a solution of 5 x SSC, 5 x Denhardt's solution, 0.5 % SDS and 100 µg/ml of denatured sonicated salmon sperm DNA for 12 hours at approximately 45°C, followed by washing twice for 30 minutes in 2 x SSC, 0.5 % SDS at a temperature of at least 65°C; which polynucleotide encodes a potassium channel subunit.

2. The isolated polynucleotide according to claim 1, wherein said hybridization is followed by washing twice for 30 minutes in 2 x SSC, 0.5 % SDS, at a temperature of at least at least 70°C.

3. The isolated polynucleotide according to claim 1, wherein said hybridization is followed by washing twice for 30 minutes in 2 x SSC, 0.5 % SDS, at a temperature of at least at least 75°C.

4. The isolated polynucleotide according to any one of claims 1-3, showing 90% identity to the polynucleotide sequence presented as SEQ ID NO: 1.

5. The isolated polynucleotide according to claim 4, showing at least 95% identity to the polynucleotide sequence presented as SEQ ID NO: 1.

6. The isolated polynucleotide according to claim 1, having the polynucleotide sequence presented as SEQ ID NO: 1.

7. The isolated polynucleotide according to claim 1, encoding a human, rat or murine potassium channel subunit.

8. The isolated polynucleotide according to claim 1, encoding the KCNQ5 potassium channel subunit having the amino acid sequence represented by SEQ ID NO: 2.

9. The isolated polynucleotide according to claim 1, encoding a KCNQ5 variant, which variant has an amino acid sequence that has been changed at one or more positions located in the conserved regions, as defined by Table 1.

10. A vector construct comprising the polynucleotide according to any of claims 1-9.

11. A recombinantly produced polypeptide encoded by the polynucleotide according to claims 1-9.

12. The polypeptide according to claim 11, being a KCNQ5 potassium channel subunit having the amino acid sequence presented as SEQ ID No. 2.

13. The polypeptide of either of claims 11-12, comprising six transmembrane domains, a P-loop, and a carboxy-terminal conserved cytoplasmic region (the "A-domain").

14. The polypeptide according to claim 11, being a KCNQ5 variant, which variant has an amino acid sequence that has been changed at one or more positions located in the conserved regions, as defined by Table 1.

15. A cell genetically manipulated by the incorporation of a heterologous polynucleotide according to any of claims 1-9, or a vector construct according to claim 10.

16. The cell according to claim 15, genetically manipulated by the incorporation of a KCNQ5 channel subunit having the amino acid sequence presented as SEQ ID NO: 2.

17. The cell according to claim 15, genetically manipulated by the incorporation of a KCNQ5 variant, which variant has an amino acid sequence that has been changed at one or more positions located in the conserved regions, as defined by Table 1.

18. The cell according to any of claims 15-17, genetically manipulated to co-express one or more KCNQ channel subunits.

19. The cell according to claim 18, genetically manipulated in order to co-express
KCNQ5 and KCNQ1 channel subunits;
KCNQ5 and KCNQ2 channel subunits;
KCNQ5 and KCNQ3 channel subunits;
KCNQ5 and KCNQ4 channel subunits;
KCNQ5 and KCNQ1 and KCNQ2 channel subunits;
KCNQ5 and KCNQ1 and KCNQ3 channel subunits;
KCNQ5 and KCNQ1 and KCNQ4 channel subunits;
KCNQ5 and KCNQ2 and KCNQ3 channel subunits;
KCNQ5 and KCNQ2 and KCNQ4 channel subunits;
KCNQ5 and KCNQ3 and KCNQ4 channel subunits;
KCNQ5 and KCNQ1 and KCNQ2 and KCNQ3 channel subunits;
KCNQ5 and KCNQ1 and KCNQ2 and KCNQ4 channel subunits;
KCNQ5 and KCNQ1 and KCNQ3 and KCNQ4 channel subunits, or
KCNQ5 and KCNQ2 and KCNQ3 and KCNQ4 channel subunits.

20. The cell according to claim 18, genetically manipulated to co-express KCNQ2 or KCNQ3, and KCNQ5 channel subunits.

21. The cell according to any of claims 15-20, being an eukaryotic cell, in particular a mammalian cell, an oocyte, or a yeast cell.

22. The cell according to claim 21, being a human embryonic kidney (HEK) cell, a HEK 293 cell, a BHK21 cell, a Chinese hamster ovary (CHO) cell, a *Xenopus laevis* oocyte (XLO) cell, a COS cell, or any other cell line able to express KCNQ potassium channels.

23. A method for obtaining a substantially homogeneous source of a human potassium channel, comprising a KCNQ5 subunit, which method comprises the steps of culturing a cellular host having incorporated expressibly therein a polynucleotide according to any of claims 1-9, or a vector construct according to claim 10, and then recovering the cultured cells.

24. The method of claim 23, comprising the subsequent step of obtaining a membrane preparation from the cultured cells.

25. A method of screening a chemical compound for capability of binding to a potassium channel comprising at least one KCNQ5 channel subunit, which method comprises the steps of
(i) subjecting the KCNQ5 channel subunit containing cell of claims 15-22 to the action of a KCNQ5 binding agent to form a complex with the KCNQ5 channel subunit containing cell;
(ii) subjecting the complex of step (i) to the action of the chemical compound to be tested; and
(iii) detecting the displacement of the KCNQ5 binding agent from the complex with the KCNQ5 channel subunit containing cell.

26. The method of claim 25, in which the KCNQ5 binding agent is
(i) radioactively labelled 1,3-dihydro-1-phenyl-3,3-bis-(4-pyridylmethyl)-2H-indol-2-one (Linopirdine); or
(ii) radioactively labelled 10,10-bis-(4-pyridinyl-methyl)-9-(10H)-anthra-cenone (XE991).

27. The method of claim 26, which compounds have been marked with ³H.

28. The method of either of claims 26-27, wherein the displacement of the KCNQ5 binding agent from the complex with the KCNQ5 channel subunit containing cell is detected by measuring the amount of radioactivity by conventional liquid scintillation counting.

29. A method of screening a chemical compound for activity on a potassium channel comprising at least one KCNQ5 channel subunit, which method comprises the steps of
(i) subjecting the KCNQ5 channel subunit containing cell according to claims 15-22 to the action of the chemical compound; and
(ii) monitoring the membrane potential, the current, the potassium flux, or the secondary calcium influx of the KCNQ5 channel subunit containing cell.

30. The method of claim 29, wherein monitoring of the membrane potential of the KCNQ5 channel subunit containing cell is performed by patch clamp techniques.

31. The method of either of claims 29-30, wherein monitoring of the membrane potential of the KCNQ5 channel subunit containing cell is performed using fluorescence methods.

32. Use of a polynucleotide sequence according to any of claims 1-9, or a vector construct according to claim 10, for the screening of genetic materials collected from mammalian tissues, in particular human tissues, for individuals having mutations in this gene.

33. A transgenic animal other than a human, comprising a knock-out mutation of the endogenous *KCNQ5* gene according to claims 1-8, or a mutated *KCNQ5* gene according to claim 9, or genetically manipulated in order to over-express the *KCNQ5* gene according to claims 1-8, or to over-express the mutated *KCNQ5* gene according to claim 9.

34. The transgenic animal according to claim 33, being a knock-out animal in which the gene is totally deleted in a homozygous state.

35. The transgenic animal according to claim 34, comprising a mutated *KCNQ5* gene according to claim 9.

36. The transgenic animal according to any of claims 33-35, being a transgenic rodent, in particular a hamster, a guinea pig, a rabbit, or a rat, a transgenic pig, a transgenic cattle, a transgenic sheep, or a transgenic goat.

## Patentansprüche

1. Isoliertes Polynucleotid mit einer Nucleinsäuresequenz, die fähig ist, unter Bedingungen von mindestens mittlerer/hoher Stringenz mit der als SEQ ID NO: 1 vorgelegten Polynucleotidsequenz oder deren komplementärem Strang zu hybridisieren, wobei die Hybridisierung in einer Lösung von 5 x SSC, 5 x Denhardt-Lösung, 0,5 % SDS und 100 µg/ml denaturierter, sonifizierter Lachssperma-DNA 12 Stunden lang bei ungefähr 45°C stattfindet, gefolgt von zweimaligem, 30 Minuten langem Spülen in 2 x SSC, 0,5 % SDS bei einer Temperatur von mindestens 65°C; wobei das Polynucleotid eine Kaliumkanaluntereinheit kodiert.

2. Isoliertes Polynucleotid nach Anspruch 1, wobei auf die Hybridisierung zweimaliges, 30 Minuten langes Spülen in 2 x SSC, 0,5 % SDS bei einer Temperatur von mindestens 70°C folgt.

3. Isoliertes Polynucleotid nach Anspruch 1, wobei auf die Hybridisierung zweimaliges, 30 Minuten langes Spülen in 2 x SSC, 0,5 % SDS bei einer Temperatur von mindestens 75°C folgt.

4. Isoliertes Polynucleotid nach einem der Ansprüche 1 - 3, das 90 % Identität mit der als SEQ ID NO:1 vorgelegten Polynucleotidsequenz zeigt.

5. Isoliertes Polynucleotid nach Anspruch 4, das mindestens 95 % Identität mit der als SEQ ID NO: 1 vorgelegten Polynucleotidsequenz zeigt.

6. Isoliertes Polynucleotid nach Anspruch 1 mit der als SEQ ID NO: 1 vorgelegten Polynucleotidsequenz.

7. Isoliertes Polynucleotid nach Anspruch 1, das eine Kaliumkanaluntereinheit von Mensch, Ratte oder Maus kodiert.

8. Isoliertes Polynucleotid nach Anspruch 1, das die Kaliumkanaluntereinheit KCNQ5 mit der durch SEQ ID NO: 2 dargestellten Aminosäuresequenz kodiert.

9. Isoliertes Polynucleotid nach Anspruch 1, das eine KCNQ5-Variante kodiert, wobei die Variante eine Aminosäuresequenz aufweist, die an einer oder mehreren Positionen, die in den wie durch Tabelle 1 definierten konservierten Regionen liegen, verändert worden ist.

10. Vektorkonstrukt, umfassend das Polynucleotid nach einem der Ansprüche 1 - 9.

11. Rekombinant hergestelltes Polypeptid, das durch das Polynucleotid nach den Ansprüchen 1 - 9 kodiert wird.

12. Polypeptid nach Anspruch 11, bei dem es sich um eine KCNQ5-Kaliumkanaluntereinheit mit der als SEQ ID NO: 2 vorgelegten Aminosäuresequenz handelt.

13. Polypeptid nach einem der beiden Ansprüche 11 - 12, umfassend sechs Transmembrandomänen, eine P-Schleife und eine carboxylterminale konservierte cytoplasmatische Region (die "A-Domäne").

14. Polypeptid nach Anspruch 11, bei dem es sich um eine KCNQ5-Variante handelt, wobei die Variante eine Aminosäuresequenz aufweist, die an einer oder mehreren Positionen, die in den wie durch Tabelle 1 definierten konservierten Regionen liegen, verändert worden ist.

15. Zelle, die durch den Einbau eines heterologen Polynucleotids nach einem der Ansprüche 1 - 9 oder eines Vektorkonstrukts nach Anspruch 10 genetisch manipuliert ist.

16. Zelle nach Anspruch 15, die durch den Einbau einer KCNQ5-Kaliumkanaluntereinheit mit der als SEQ ID NO: 2 vorgelegten Aminosäuresequenz genetisch manipuliert ist.

17. Zelle nach Anspruch 15, die durch den Einbau einer KCNQ5-Variante genetisch manipuliert ist, wobei die Variante eine Aminosäuresequenz aufweist, die an einer oder mehreren Positionen, die in den wie durch Tabelle 1 definierten konservierten Regionen liegen, verändert worden ist.

18. Zelle nach einem der Ansprüche 15 - 17, die genetisch manipuliert ist, um eine oder mehrere KCNQ-Kanaluntereinheiten zu koexprimieren.

19. Zelle nach Anspruch 18, die genetisch manipuliert ist, um
die Kanaluntereinheiten KCNQ5 und KCNQ1,
die Kanaluntereinheiten KCNQ5 und KCNQ2,
die Kanaluntereinheiten KCNQ5 und KCNQ3,
die Kanaluntereinheiten KCNQ5 und KCNQ4,
die Kanaluntereinheiten KCNQ5 und KCNQ1 und KCNQ2,
die Kanaluntereinheiten KCNQ5 und KCNQ1 und KCNQ3,
die Kanaluntereinheiten KCNQ5 und KCNQ1 und KCNQ4,
die Kanaluntereinheiten KCNQ5 und KCNQ2 und KCNQ3,
die Kanaluntereinheiten KCNQ5 und KCNQ2 und KCNQ4,
die Kanaluntereinheiten KCNQ5 und KCNQ3 und KCNQ4,
die Kanaluntereinheiten KCNQ5 und KCNQ1 und KCNQ2 und KCNQ3,
die Kanaluntereinheiten KCNQ5 und KCNQ1 und KCNQ2 und KCNQ4,
die Kanaluntereinheiten KCNQ5 und KCNQ1 und KCNQ3 und KCNQ4 oder
die Kanaluntereinheiten KCNQ5 und KCNQ2 und KCNQ3 und KCNQ4
zu koexprimieren.

20. Zelle nach Anspruch 18, die genetisch manipuliert ist, um die Kanaluntereinheiten KCNQ2 oder KCNQ3 und KCNQ5 zu koexprimieren.

21. Zelle nach einem der Ansprüche 15 - 20, bei der es sich um eine eukaryotische Zelle, insbesondere eine Säugerzelle, eine Oocyte oder eine Hefezelle handelt.

22. Zelle nach Anspruch 21, bei der es sich um eine menschliche embryonale Nierenzelle (HEK-Zelle), eine HEK 293-Zelle, eine BHK21-Zelle, eine Eierstockzelle des chinesischen Hamsters (CHO-Zelle), eine Oocytenzelle aus *Xaenopus laevis* (XLO-Zelle), eine COS-Zelle oder jede andere Zelllinie handelt, die in der Lage ist, KCNQ-Kaliumkanäle zu exprimieren.

23. Verfahren zum Erhalten einer im Wesentlichen homogenen Quelle eines menschlichen Kaliumkanals, der eine KCNQ5-Untereinheit umfasst, wobei das Verfahren die Schritte umfasst, einen zellulären Wirt, der ein Polynucleotid nach einem der Ansprüche 1 - 9 oder ein Vektorkonstrukt nach Anspruch 10 exprimierbar darin eingebaut hat, zu züchten und dann die gezüchteten Zellen zu gewinnen.

24. Verfahren nach Anspruch 23, umfassend den anschließenden Schritt, von den gezüchteten Zellen ein Membranpräparat zu erhalten.

25. Verfahren zum Durchmustern einer chemischen Verbindung auf die Fähigkeit hin, an einen Kaliumkanal zu binden, der mindestens eine KCNQ5-Kanaluntereinheit umfasst, wobei das Verfahren die Schritte umfasst,
(i) die Zelle nach den Ansprüchen 15-22, die eine KCNQ5-Kanaluntereinheit enthält, der Wirkung eines KCNQ5 bindenden Mittels zu unterwerfen, um einen Komplex mit der Zelle zu bilden, die eine KCNQ5-Kanaluntereinheit enthält,
(ii) den Komplex von Schritt (i) der Wirkung der zu testenden chemischen Verbindung zu unterwerfen und
(iii) die Verdrängung des KCNQ5 bindenden Mittels aus dem Komplex mit der Zelle, die eine KCNQ5-Kanaluntereinheit enthält, nachzuweisen.

26. Verfahren nach Anspruch 25, wobei es sich bei dem KCNQ5 bindenden Mittel um
(i) radioaktiv markiertes 1,3-Dihydro-1-phenyl-3,3-bis-(4-pyridylmethyl)-2H-indol-2-on (Linopirdin) oder
(ii) radioaktiv markiertes 10,10-Bis-(4-pyridinyl-methyl)-9-(10H)-anthracenon (XE991)
handelt.

27. Verfahren nach Anspruch 26, wobei die Verbindungen mit ³H markiert worden sind.

28. Verfahren nach einem der beiden Ansprüche 26 - 27, wobei die Verdrängung des KCNQ5 bindenden Mittels aus dem Komplex mit der Zelle, die eine KCNQ5-Kanaluntereinheit enthält, durch Messen der Menge an Radioaktivität durch herkömmliche Flüssigszintillationszählung nachgewiesen wird.

29. Verfahren zum Durchmustern einer chemischen Verbindung auf eine Aktivität auf einen Kaliumkanal hin, der mindestens eine KCNQ5-Kanaluntereinheit umfasst, wobei das Verfahren die Schritte umfasst,
(i) die Zelle nach den Ansprüchen 15 - 22, die eine KCNQ5-Kanaluntereinheit enthält, der Wirkung der chemischen Verbindung zu unterwerfen und
(ii) das Membranpotenzial, den Strom, den Kaliumfluss oder den sekundären Calciumzufluss der Zelle, die eine KCNQ5-Kanaluntereinheit enthält, zu überwachen.

30. Verfahren nach Anspruch 29, wobei das Überwachen des Membranpotenzials der Zelle, die eine KCNQ5-Kanaluntereinheit enthält, durch Patch-Clamp-Techniken durchgeführt wird.

31. Verfahren nach einem der beiden Ansprüche 29 - 30, wobei das Überwachen des Membranpotenzials der Zelle, die eine KCNQ5-Kanaluntereinheit enthält, unter Verwendung von Fluoreszenzverfahren durchgeführt wird.

32. Verwendung einer Polynucleotidsequenz nach einem der Ansprüche 1 - 9 oder eines Vektorkonstrukts nach Anspruch 10 zum Durchmustern von genetischen Materialien, die aus Säugergeweben, insbesondere menschlichen Geweben gewonnen sind, auf Individuen mit Mutationen in diesem Gen.

33. Transgenes Tier, bei dem es sich nicht um einen Menschen handelt, das eine Knockout-Mutation des endogenen Gens *KCNQ5* nach den Ansprüchen 1 - 8 oder ein mutiertes Gen *KCNQ5* nach Anspruch 9 umfasst oder genetisch manipuliert ist, um das Gen *KCNQ5* nach den Ansprüchen 1 - 8 zu überexprimieren oder um das mutierte Gen *KCNQ5* nach Anspruch 9 zu überexprimieren.

34. Transgenes Tier nach Anspruch 33, bei dem es sich um ein Knockout-Tier handelt, bei dem das Gen in einem homozygoten Zustand vollständig deletiert ist.

35. Transgenes Tier nach Anspruch 34, umfassend ein mutiertes Gen *KCNQ5* nach Anspruch 9.

36. Transgenes Tier nach einem der Ansprüche 33 - 35, bei dem es sich um ein transgenes Nagetier, insbesondere einen Hamster, ein Meerschweinchen, ein Kaninchen oder eine Ratte, ein transgenes Schwein, ein transgenes Rind, ein transgenes Schaf oder eine transgene Ziege handelt.

## Revendications

1. Polynucléotide isolé ayant une séquence d'acide nucléique qui est capable de s'hybrider dans des conditions de stringence au moins moyennes/élevées à la séquence polynucléotidique représentée par SEQ ID NO: 1, ou son brin complémentaire, dans lequel ladite hybridation se produit dans une solution de SSC 5X, une solution de Denhardt 5X, 0,5 % de SDS et 100 µg/ml d'ADN de sperme de saumon soniqué dénaturé pendant 12 heures à approximativement 45°C, suivie d'un lavage deux fois pendant 30 minutes dans du SSC 2X, 0,5 % de SDS à une température d'au moins 65°C, lequel polynucléotide code pour une sous-unité de canal potassique.

2. Polynucléotide isolé selon la revendication 1, dans lequel ladite hybridation est suivie d'un lavage deux fois pendant 30 minutes dans du SSC 2X, 0,5 % de SDS, à une température d'au moins 70°C.

3. Polynucléotide isolé selon la revendication 1, dans lequel ladite hybridation est suivie d'un lavage deux fois pendant 30 minutes dans du SSC 2X, 0,5 % de SDS, à une température d'au moins 75°C.

4. Polynucléotide isolé selon l'une quelconque des revendications 1 à 3, présentant 90 % d'identité avec la séquence polynucléotidique représentée par SEQ ID NO: 1.

5. Polynucléotide isolé selon la revendication 4, présentant au moins 95 % d'identité avec la séquence polynucléotidique représentée par SEQ ID NO: 1.

6. Polynucléotide isolé selon la revendication 1, ayant la séquence polynucléotidique représentée par SEQ ID NO: 1.

7. Polynucléotide isolé selon la revendication 1, codant pour une sous-unité de canal potassique humain, de rat ou murin.

8. Polynucléotide isolé selon la revendication 1, codant pour la sous-unité du canal potassique KCNQ5 ayant la séquence d'acides aminés représentée par SEQ ID NO: 2.

9. Polynucléotide isolé selon la revendication 1, codant pour un variant de KCNQ5, lequel variant a une séquence d'acides aminés qui a été changée au niveau d'une ou plusieurs positions situées dans les régions conservées, telles que définies par le Tableau 1.

10. Vecteur comprenant le polynucléotide selon l'une quelconque des revendications 1 à 9.

11. Polypeptide produit de manière recombinante codé par le polynucléotide selon les revendications 1 à 9.

12. Polypeptide selon la revendication 11, étant une sous-unité du canal potassique KCNQ5 ayant la séquence d'acides aminés représentée par SEQ ID NO: 2.

13. Polypeptide selon l'une ou l'autre des revendications 11 et 12, comprenant six domaines transmembranaires, une boucle P, une région carboxy-terminale cytoplasmique conservée (le "domaine A").

14. Polypeptide selon la revendication 11, étant un variant de KCNQ5, lequel variant a une séquence d'acides aminés qui a été changée au niveau d'une ou plusieurs positions situées dans les régions conservées, telles que définies par le Tableau 1.

15. Cellule génétiquement manipulée par l'incorporation d'un polynucléotide hétérologue selon l'une quelconque des revendications 1 à 9, ou d'un vecteur selon la revendication 10.

16. Cellule selon la revendication 15, génétiquement manipulée par l'incorporation d'une sous-unité de canal KCNQ5 ayant la séquence d'acides aminés représentée par SEQ ID NO: 2.

17. Cellule selon la revendication 15, génétiquement manipulée par l'incorporation d'un variant de KCNQ5, lequel variant a une séquence d'acides aminés qui a été changée au niveau d'une ou plusieurs positions situées dans les régions conservées, telles que définies par le Tableau 1.

18. Cellule selon l'une quelconque des revendications 15 à 17, génétiquement manipulée pour co-exprimer une ou plusieurs sous-unités de canal KCNQ.

19. Cellule selon la revendication 18, génétiquement manipulée afin de co-exprimer
des sous-unités des canaux KCNQ5 et KCNQ1 ;
des sous-unités des canaux KCNQ5 et KCNQ2 ;
des sous-unités des canaux KCNQ5 et KCNQ3 ;
des sous-unités des canaux KCNQ5 et KCNQ4 ;
des sous-unités des canaux KCNQ5 et KCNQ1 et KCNQ2 ;
des sous-unités des canaux KCNQ5 et KCNQ1 et KCNQ3 ;
des sous-unités des canaux KCNQ5 et KCNQ1 et KCNQ4 ;
des sous-unités des canaux KCNQ5 et KCNQ2 et KCNQ3 ;
des sous-unités des canaux KCNQ5 et KCNQ2 et KCNQ4 ;
des sous-unités des canaux KCNQ5 et KCNQ3 et KCNQ4 ;
des sous-unités des canaux KCNQ5 et KCNQ1 et KCNQ2 et KCNQ3 ;
des sous-unités des canaux KCNQ5 et KCNQ1 et KCNQ2 et KCNQ4 ;
des sous-unités des canaux KCNQ5 et KCNQ1 et KCNQ3 et KCNQ4, ou
des sous-unités des canaux KCNQ5 et KCNQ2 et KCNQ3 et KCNQ4.

20. Cellule selon la revendication 18, génétiquement manipulée pour co-exprimer des sous-unités des canaux KCNQ2 ou KCNQ3, et KCNQ5.

21. Cellule selon l'une quelconque des revendications 15 à 20, étant une cellule eucaryote, en particulier une cellule de mammifères, un oocyte ou une cellule de levure.

22. Cellule selon la revendication 21, étant une cellule de rein embryonnaire humain (HEK), une cellule HEK 293, une cellule BHK21, une cellule d'ovaire de hamster chinois (CHO), une cellule d'oocyte de *Xenopus laevis* (XLO), une cellule COS, ou toute autre lignée cellulaire capable d'exprimer les canaux potassiques KCNQ.

23. Procédé d'obtention d'une source pratiquement homogène d'un canal potassique humain, comprenant une sous-unité KCNQ5, lequel procédé comprend les étapes consistant à cultiver un hôte cellulaire ayant incorporé à l'intérieur de manière exprimable un polynucléotide selon l'une quelconque des revendications 1 à 9, ou un vecteur selon la revendication 10, et ensuite à récupérer les cellules cultivées.

24. Procédé selon la revendication 23, comprenant l'étape suivante consistant à obtenir une préparation membranaire provenant des cellules cultivées.

25. Procédé de criblage d'un composé chimique pour sa capacité à se lier à un canal potassique comprenant au moins une sous-unité de canal KCNQ5, lequel procédé comprend les étapes consistant à
(i) soumettre la cellule contenant la sous-unité de canal KCNQ5 selon les revendications 15 à 22 à l'action d'un agent de liaison de KCNQ5 pour former un complexe avec la cellule contenant la sous-unité de canal KCNQ5 ;
(ii) soumettre le complexe de l'étape (i) à l'action du composé chimique à tester ; et
(iii) détecter le déplacement de l'agent de liaison de KCNQ5 du complexe avec la cellule contenant la sous-unité de canal KCNQ5.

26. Procédé selon la revendication 25, dans lequel l'agent de liaison de KCNQ5 est
(i) la 1,3-dihydro-1-phényl-3,3-bis-(4-pyridyl-méthyl)-2H-indol-2-one (Linopirdine) marquée de manière radioactive ; ou
(ii) la 10,10-bis-(4-pyridinyl-méthyl)-9-(10H)-anthracénone (XE991) marquée de manière radioactive.

27. Procédé selon la revendication 26, dont les composés ont été marqués au ³H.

28. Procédé selon l'une ou l'autre des revendications 26 et 27, dans lequel le déplacement de l'agent de liaison de KCNQ5 du complexe avec la cellule contenant la sous-unité de canal KCNQ5 est détecté en mesurant la quantité de radioactivité par un comptage conventionnel à scintillation liquide.

29. Procédé de criblage d'un composé chimique pour une activité sur un canal potassique comprenant au moins une sous-unité de canal KCNQ5, lequel procédé comprend les étapes consistant à
(i) soumettre la cellule contenant la sous-unité de canal KCNQ5 selon les revendications 15 à 22 à l'action du composé chimique ; et
(ii) contrôler le potentiel membranaire, le courant, le flux de potassium ou l'influx de calcium secondaire de la cellule contenant la sous-unité de canal KCNQ5.

30. Procédé selon la revendication 29, dans lequel le contrôle du potentiel membranaire de la cellule contenant la sous-unité de canal KCNQ5 est effectué par des techniques de patch-clamp.

31. Procédé selon l'une ou l'autre des revendications 29 et 30, dans lequel le contrôle du potentiel membranaire de la cellule contenant la sous-unité de canal KCNQ5 est effectué en utilisant des procédés de fluorescence.

32. Utilisation d'une séquence polynucléotidique selon l'une quelconque des revendications 1 à 9, ou d'un vecteur selon la revendication 10, pour le criblage de matériaux génétiques recueillis à partir de tissus de mammifères, en particulier de tissus humains, pour des individus présentant des mutations dans ce gène.

33. Animal transgénique autre qu'un homme, comprenant une mutation knock-out du gène *KCNQ5* endogène selon les revendications 1 à 8, ou un gène *KCNQ5* muté selon la revendication 9, ou génétiquement manipulé afin de surexprimer le gène *KCNQ5* selon les revendications 1 à 8, ou pour surexprimer le gène *KCNQ5* muté selon la revendication 9.

34. Animal transgénique selon la revendication 33, étant un animal knock-out dans lequel le gène est totalement délété dans un état homozygote.

35. Animal transgénique selon la revendication 34, comprenant un gène *KCNQ5* muté selon la revendication 9.

36. Animal transgénique selon l'une quelconque des revendications 33 à 35, étant un rongeur transgénique, en particulier un hamster, un cobaye, un lapin ou un rat, un cochon transgénique, une vache transgénique, un mouton transgénique ou une chèvre transgénique.
